(19) **Europäisches Patentamt** / **European Patent Office** / **Office européen des brevets**

(11) **EP 4 237 397 B1**

(12) # EUROPEAN PATENT SPECIFICATION

(45) Date of publication and mention
of the grant of the patent:
**15.07.2026 Bulletin 2026/29**

(21) Application number: **21810825.6**

(22) Date of filing: **25.10.2021**

(51) International Patent Classification (IPC):
**C07C 37/74** *(2006.01)*  **C07C 39/04** *(2006.01)*
**C07C 45/82** *(2006.01)*  **C07C 49/08** *(2006.01)*

(52) Cooperative Patent Classification (CPC):
(C-Sets available)
**C07C 37/74; C07C 45/82**    (Cont.)

(86) International application number:
**PCT/US2021/056457**

(87) International publication number:
**WO 2022/093692 (05.05.2022 Gazette 2022/18)**

(54) **METHOD FOR PRODUCING PHENOL**

VERFAHREN ZUR HERSTELLUNG VON PHENOL

PROCÉDÉ DE PRODUCTION DE PHÉNOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.10.2020 RU 2020135296**

(43) Date of publication of application:
**06.09.2023 Bulletin 2023/36**

(73) Proprietor: **SABIC Global Technologies B.V.**
**4612 PX Bergen op Zoom (NL)**

(72) Inventors:
• **SOKOLOV, Andrey Yurevich**
**Saint-Petersburg, 196158 (RU)**
• **SOKOLOV, Alexey Andreevich**
**10587 Berlin (DE)**
• **NELSON, Mark Erik**
**Mount Vernon, Indiana 47620 (US)**

(74) Representative: **Modiano, Gabriella Diana**
**Modiano & Partners SA**
**Steinsdorfstraße 14**
**80538 München (DE)**

(56) References cited:
**JP-A- 2000 086 559    US-A- 4 251 325**

• **ROMANOVA N A ET AL: "Structure and energy
optimization of the phenol and acetone
separation process using reaction mixture
components as entrainers", PETROLEUM
CHEMISTRY, PLEIADES PUBLISHING,
MOSCOW, vol. 57, no. 5, 30 June 2017
(2017-06-30), pages 430 - 435, XP036267982,
ISSN: 0965-5441, [retrieved on 20170630], DOI:
10.1134/S0965544117020220**

**(Cont. next page)**

(52) Cooperative Patent Classification (CPC): (Cont.)

C-Sets
**C07C 37/74, C07C 39/04;**
**C07C 45/82, C07C 49/08**

**Description**

CROSS-REFERENCE TO RELATED APPLICATION

The present application is an international filing which claims priority to Russia Patent Application 2020135296, filed October 27, 2020 and published April 27, 2022.

BACKGROUND

Phenol, produced on an industrial-scale by the acid-catalyzed cleavage/decomposition of cumene hydroperoxide (CHP), can be purified via a series of distillations in which progressively heavier components of the cleavage mixture are separated as a bottom stream. The decomposition product is neutralized before being separated into products of phenol and acetone, by-products, and unreacted cumene (CUM) for recycle. The neutralized cumene hydroperoxide cleavage products can be separated into a crude acetone fraction (overhead stream) and a crude phenol fraction (bottom stream) via a column (also known as a distillation column). Impurities such as hydroxyacetone (HA) and alpha-methylstyrene (AMS) can be removed in the overhead stream, with some AMS being recovered in the bottom stream.

Many phenol production plants will overload the first column (e.g., distillation column) which separates neutralized cumene hydroperoxide cleavage products into a crude acetone fraction (overhead stream) and a crude phenol fraction (bottom stream), in an attempt to increase the output of the column. However, this practice can disrupt the mass-balance and the efficiency of the first column, thus leading to an increase in unwanted hydroxyacetone and alpha-methylstyrene impurities in the crude phenol product stream. After the first column there can be several (e.g., three) additional columns. The produced phenol can be used in the production of bis-phenol A (BPA). Desirably, the BPA production uses reactants with very low impurities. HA is known to have adverse impacts on color and quality of BPA, which can lead to poor polycarbonate (PC) color.

Accordingly, the practice of overloading the distillation column can significantly reduce the quality and commercial value of the final phenol product. Impurities can also continue to form in the phenol product even after production. For example, the impurity 2-methylbenofuran (2-MBF) is formed via the interaction of hydroxyacetone with phenol and is extremely difficult to remove.

Hydroxyacetone removal methods can include both physical (for example, rectification), and chemical (for example, addition of chemical reagents), both of which are costly and increase the complexity of the phenol purification system. Accordingly, such removal methods should be avoided if possible. The quality of the hydroxyacetone removal can also depend on many factors, for example, crude phenol composition, process technical parameters, equipment, or the efficiency of mass-transfer devices.

U.S. Patent No. 3,405,038 describes a method of hydroxyacetone removal from a neutralized cumene hydroperoxide cleavage mixture using azeotrope rectification. Hydroxyacetone forms a minimal azeotrope with cumene. According to the method of U.S. Patent No. 3,405,038, effective hydroxyacetone removal from a crude phenol stream requires a feed ratio of at least 0.28 parts by weight of cumene to one part of phenol. If the initial feed mass ratio is lower, additional cumene is required. Moreover, above the feed point, the temperature is maintained from 110 °C to the cumene boiling point. In the examples, the hydroxyacetone content in the bottoms varies from 30 to 65 ppm. However, these levels of hydroxyacetone are generally unacceptable for modern phenol production. In addition, this process requires constant recycling of substantial amounts of cumene; this is expensive for both capital and utility usage.

U.S. Patent No. 4,251,325 discloses that phenol containing less than 30 ppm of hydroxyacetone can be produced from the crude phenol fraction after the neutralized cleavage cumene hydroperoxide mixture is separated in a distillation column in which acetone and water are separated from the phenol fraction. In an additional subsequent distillation column, the rectification is maintained with a combination of cumene and/or alpha-methylstyrene being up to 22.0 wt%. The combination feed containing AMS and/or cumene is fed to an intermediate point in the subsequent distillation column; an overhead fraction containing cumene and alpha-methylstyrene and a substantial fraction of the hydroxyacetone present in the feed is removed as an overhead product. The phenol fraction containing less than 30 ppm of hydroxyacetone is removed from the column bottom. By adjusting temperature and pressure, the column is controlled such that the cumene and/or alpha-methylstyrene forms from between 55 and 80 wt% of the composition on the uppermost 15 to 70% of the trays in the stripping section, and phenol forms more than 50 wt% of the composition on the bottom most 10 to 50% of the trays in the stripping section. This method is a step forward in comparison with U.S. Patent No. 3,405,038, however it has a number of disadvantages. For example, the implementation of this method requires an additional column using a very high reflux ratio (e.g., from 17 to 29) which equates to both high capital and high operating expenses.

In Russian (RU) Patent No. 2,323,202, after the neutralized cumene hydroperoxide cleavage products are separated into a crude acetone fraction and a crude phenol fraction, the removal of hydroxyacetone from the crude phenol fraction that is recovered is described using azeotropic-extractive rectification with a separating agent. The separating agent includes a hydrocarbon (cumene and/or alpha-methylstyrene) as one component, and water as the second component. RU Patent No. 2,323,202 involves feeding a separating agent or agents along with the crude phenol to a column, maintaining the mass ratio of hydrocarbon and water such that it is equal to or is above the mass ratio of hydrocarbon and water in the corresponding azeotrope mixture. The hydroxyacetone separating agents are removed with the distillate; and the distillate organic phase is used for reflux. Significant disadvantages of this method include a high reflux ratio varied from two to three and corresponding high energy consumption, and increased phenol content up to 3% in the aqueous phase. Removal of this phenol will load the de-phenolation area of the plant. Additionally, this method is not very efficient.

Other methods utilized to reduce hydroxyacetone are, to varying degrees, capital intensive and have elevated operating costs.

SUMMARY

Disclosed, in various embodiments of the present disclosure, are systems and methods of producing phenol, and systems and methods of separating phenol from a phenol product stream.

There is a need for a method of phenol production that can avoid the problem of overloading the distillation column (e.g., crude acetone distillation column), thus maintaining the efficiency of the column and greatly reducing impurity levels in the phenol product.

The instant invention is thus directed to a method of separating phenol comprising: separating a first portion of acetone from a product stream in an evaporator unit, wherein the first portion of the acetone includes recycle acetone and bypass acetone; recycling the recycle acetone; withdrawing a bottom fraction from the evaporator unit; neutralizing the bottom fraction to form a distillation feed stream that is directed to a distillation column; separating the distillation feed stream into a bottom stream and an overhead stream, the bottom stream including a crude phenol fraction; passing the overhead stream through a condenser to produce a distillate as a crude acetone fraction, and wherein a reflux ratio of the distillation column is less than or equal to 0.40, with the reflux ratio a ratio of a weight of a reflux to distillate weight; and directing the bypass acetone around the distillation column, wherein the product stream is a cumene hydroperoxide cleavage product stream; and wherein the recycle acetone is recycled to a cumene hydroperoxide cleavage.

The above described and other features are exemplified by the following figures and detailed description.

Any combination or permutation of embodiments is envisioned. Additional advantageous features, functions and applications of the disclosed systems and methods of the present disclosure will be apparent from the description which follows, particularly when read in conjunction with the appended figures.

BRIEF DESCRIPTION OF THE DRAWINGS

The following is a brief description of the drawings wherein like elements are numbered alike and which are presented for the purposes of illustrating the exemplary embodiments disclosed herein and not for the purposes of limiting the same.

Exemplary embodiments of the present disclosure are further described with reference to the appended figures. It is to be noted that the various steps, features and combinations of steps/features described below and illustrated in the figures can be arranged and organized differently to result in embodiments which are still within the scope of the present disclosure. To assist those of ordinary skill in the art in making and using the disclosed systems and methods, reference is made to the appended figures, wherein:

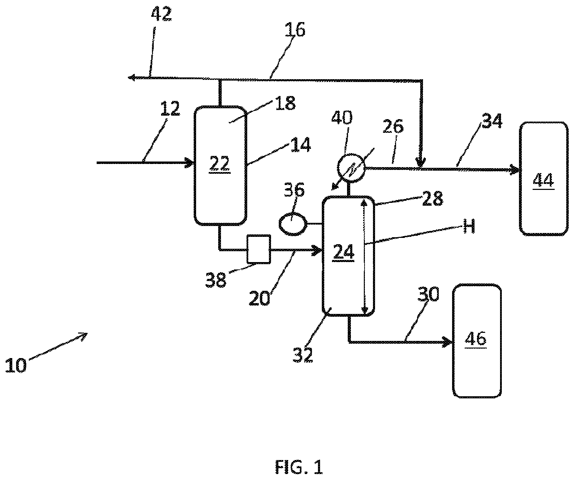

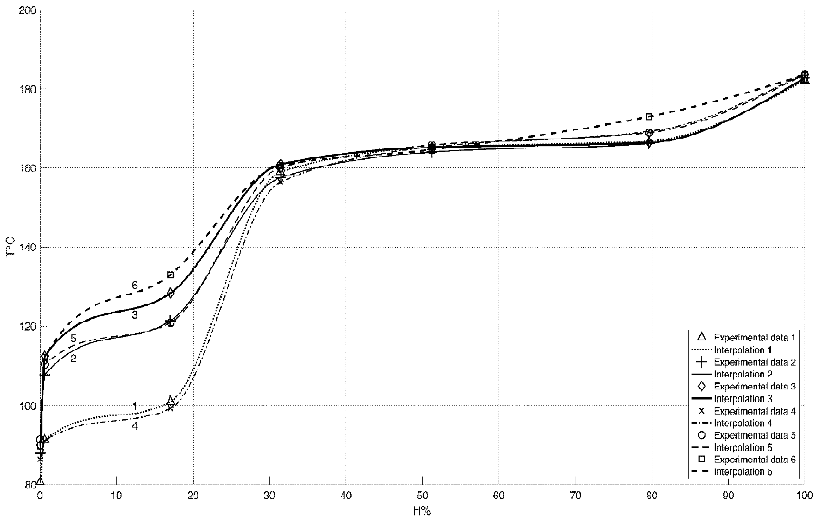

FIG. 1 is a schematic diagram representing a system configuration for use in a method for producing phenol and/or for separating phenol from a product stream.

FIG. 2 is a plotted line graph representing temperature profile data from a column for use in a method of producing phenol and/or for separating phenol from a product stream; Fig. 2 illustrates a determined temperature profile for hydroxyacetone removal (lines 1 to 3) and for hydroxyacetone and alpha-methylstyrene removal (lines 4 to 6) at a feed temperature of 100 °C, cumene to water weight ratio of 0.80 to 1.20 (lines 1, 4), 1.20 to 1.45 (lines 2, 5), and 1.45 to 1.70 (lines 3, 6).

DETAILED DESCRIPTION

The present invention provides advantageous methods for producing phenol, and improved methods for separating phenol from a product stream (e.g., from a phenol product stream).

As noted, neutralized cumene hydroperoxide cleavage products can be separated into a crude acetone fraction (overhead stream) and a crude phenol fraction (bottom stream) via a column (e.g., a distillation column). In general, the capacity or throughput of a distillation column is determined mainly by the designed vapor stream (load) or the overhead (OVHD), which moves upwards in the column, and is enriched (e.g., maximally enriched) by the volatile component at the top of the column. The overhead stream emerging from the top of the distillation column, after condensation in a condenser, can be divided into a distillate which is obtained as the overhead product (e.g., the crude acetone fraction) and into a reflux (which can be returned back into the distillation column and which has the same general composition as the distillate). The ratio of reflux to distillate is called the reflux ratio (R). A decrease in reflux leads to a decrease of the reflux ratio (R). Therefore, a decrease in reflux at constant distillation feed stream feed rate (capacity) and distillate results in overhead volume reduction (volumetric flow rate reduction), and a corresponding decrease in energy consumption of the process (reboiler duty). Reducing reflux or reflux ratio (R) at constant overhead, with other column conditions equal, allows an increase in the feed rate (capacity) of the distillation column. This increase in the feed rate (capacity) of the distillation column occurs because according to the total material balance of the column, the feed is divided into the overhead product selected as distillate and the bottom product selected as the bottom stream, thereby simultaneously increasing the amount of distillate (crude acetone fraction) and bottom product (crude phenol fraction). Thus, a noticeable decrease of reflux (R) leads to a noticeable decrease of overhead and allows, depending on the assigned tasks, to noticeably reduce the energy consumption of the process and/or to increase the capacity of the distillation column (e.g., to increase the production of phenol and/or acetone products or simultaneously in one degree or another to solve both of these problems).

A method disclosed herein for separating phenol from a phenol product stream can avoid the problem of overloading the distillation column, thus maintaining the efficiency of the column and greatly reducing impurity levels in the phenol product. The present method can reduce the load on the distillation column and can increase the efficiency of hydroxyacetone removal from the crude phenol fraction. To accomplish this, two main factors can be employed. First, the phenol product stream (e.g., from the cumene hydroperoxide decomposition stage of phenol production) is introduced to an evaporator unit where acetone is evaporated (e.g., flash evaporated via a flash evaporator unit) and thereby removed from the phenol product stream. A portion of the removed acetone can be recycled back to the cumene hydroperoxide decomposition stage as recycled acetone. The remainder of the removed acetone can bypass the distillation column and be directed to an acetone purification stage (e.g., acetone purification train) and/or can be combined with acetone (crude acetone fraction) exiting the distillation column. The combined stream can be directed to an acetone purification stage. This process of employing the bypass acetone stream (e.g., with all other conditions being equal) due to decreasing mainly acetone content in the raw feed, allows not only a volumetric flow rate reduction of 8 to 17% in the overhead stream from the distillation column, and/or a distillation column volumetric feed flow rate reduction of 6 to 13% to the distillation column, but also (due to the increase of cumene to water weight ratio in the column) increases the efficiency of hydroxyacetone removal from the crude phenol fraction. Second, maintaining a determined temperature profile in the distillation column (e.g., along a section of the distillation column) allows a marked reduction in the reflux ratio from a range of 0.5 to 0.8 down to less than 0.5 (e.g., down to less than or equal to 0.40; down to 0 to 0.1). A simulation mass balance of the distillation column for separating a neutralized cleavage mixture in a conventional phenol plant allows one to calculate changes in the main streams, for example, such as overhead (OVHD) and the feed caused using recycle (bypass) acetone from the evaporator unit around the distillation column directly to the acetone purification train. Simulations were carried out taking into account feed rate reduction caused by the use of the set point recycle and constancy of the reflux ratio both without and with recycle. According to simulations (the calculation results), the use of 25% acetone recycle (from the total acetone recycle from the evaporation unit) in the form of a bypass allowed a reduction of the overhead stream by at least 8.5% and at least 17% when using 50% acetone recycle from the evaporator unit. The determined temperature profile is a temperature profile along the height of the column that increases hydroxyacetone removal (e.g., greater than 98%) from the bottom (crude phenol fraction) of the distillation column.

Thus, the use of both of these factors simultaneously that is: (i) the reduced reflux ratio with the bypass of the bypass acetone (e.g., light acetone fraction) from the evaporator unit; and (ii) maintaining a determined temperature profile in the distillation column, with the other conditions being equal (e.g., unchanged) allows a reduced overhead stream volumetric flow rate and/or an increased volumetric feed rate (capacity) of the distillation column by at least about 30 to 40%. As will be shown below in Table 3, lowering only the reflux ratio (without using a recycle) from 0.5 to 0.1, and to about 0 allows one to reduce the overhead stream by at least 24 and 30%, respectively. Simultaneous reduction of reflux ratio and the use of recycle (bypass) of 50% allows further reduction of the overhead stream, at least to 30% and 40 % respectively. A decrease in the initial value of the reflux ratio from about 0.6-0.8 (generally accepted by some phenol plants in the industry) to 0.1 to 0 leads to an even greater effect of reducing the overhead stream. For example and as discussed further below, the volumetric flow rate of the overhead stream of the distillation column of the present disclosure can be reduced by greater than 25%, such as greater than 35%, as compared to a distillation column having a reflux ratio of greater than or equal to 0.5 and/or without selection/bypass of at least 25 vol% of a bypass stream from the evaporator unit. As will be shown below in Table 3, lowering only the reflux ratio (without using a recycle) from 0.5 to 0.1, and to about 0 allows one to reduce the overhead stream by at least 24 and 30%, respectively. Simultaneous reduction of reflux ratio and the use of recycle (bypass) of 25% allows a further reduction of the overhead stream, at least to 25% and 35% respectively. Moreover, the volumetric flow rate of the distillation feed stream of the distillation column of the present disclosure can be increased by greater than 30%, such as greater than 40%, as compared to a distillation column having a reflux ratio of greater than or equal to 0.5 and/or without selection/bypass of at least 50 vol% of a bypass stream from the evaporator unit.

In addition, using the determined temperature profile, regardless of the cumene to water weight ratio varied from 0.8 to 1.7, this thereby results in improved hydroxyacetone removal from the bottom stream (e.g., from the crude phenol stream/fraction).

With this process/method, the crude phenol stream/fraction (e.g., exiting the distillation column) can comprise less than or equal to 25 ppm hydroxyacetone, for example, less than or equal to 10 ppm, even more particularly, less than or equal to 5 ppm. Furthermore, by increasing the temperature in the bottom stream (e.g., especially in the lower part of the distillation column), the crude phenol stream/fraction can comprise less than or equal to 0.20 wt% of alpha-methylstyrene, for example, less than or equal to 0.10 wt%, or less than or equal to 0.05 wt%, or less than or equal to 0.01 wt%.

Greater than or equal to 25 vol% of the acetone from the top of the evaporator flash unit can be directed as bypass acetone around the distillation column directly to acetone purification (e.g., to an acetone purification stage/train) and/or combined with the crude acetone fraction; such as greater than or equal to 50 vol% of the acetone from the top of the evaporator flash unit. As such, the volumetric flow rate of the overhead stream of the distillation column of the present disclosure can be reduced by greater than or equal to 15%, such as greater than or equal to 25%, as compared to a distillation column having a reflux ratio of greater than or equal to 0.25 and without selection/bypass of at least 25 vol% of a bypass stream from an evaporator unit. The volumetric flow rate of the overhead stream of the distillation column can be reduced by greater than 25%, such as greater than 35%, as compared to a distillation column having a reflux ratio of greater than or equal to 0.5 and without selection/bypass of at least 25 vol% of a bypass stream from an evaporator unit.

In accordance with the present method, an evaporator unit feed stream (e.g., phenol product stream) can be passed through an evaporator unit. The evaporator unit feed stream (e.g., phenol product stream) can comprise cleaved/decomposed cumene hydroperoxide. For example, the evaporator unit feed stream (e.g., phenol product stream) can comprise phenol, acetone, water, cumene, alpha-methylstyrene, hydroxyacetone, 2-methylbenofuran, or a combination comprising at least one of the foregoing. The evaporator unit can be a flash evaporator unit, for example, a re-purposed cumene hydroperoxide decomposition reactor, a short column, or another type of evaporator. A bypass stream comprising acetone can be withdrawn from a top portion of the evaporator unit as bypass/recycle acetone.

A bottom fraction comprising acetone and phenol can be withdrawn from a bottom portion of the evaporator unit. The bottom fraction can be neutralized, for example, with base reagent, such as with caustic or in an alkaline hydroxide, to form the distillation feed stream. For example, the distillation feed stream can comprise 10 wt% to 20 wt% cumene and/or alpha-methylstyrene, e.g., 11 wt% to 16 wt%, or 12 wt% to 15 wt%. The distillation feed stream can also comprise less than or equal to 2,200 ppm of hydroxyacetone, for example, 500 to 1,700 ppm, or, 1,000 to 1,400 ppm. The distillation feed stream can further comprise a cumene to water weight ratio of 0.5 to 2, for example, 0.8 to 1.7, or 1.3 to 1.6. A temperature of the distillation feed stream can be 60 °C to 120 °C, for example, 75 °C to 110 °C, or 95 °C to 110 °C, e.g., 100 °C.

The distillation feed stream can be passed through the distillation column. The distillation column, for example, can be a packed bed column or a column with internal trays. The distillation column includes a rectifying section and a stripping section. In a non-limiting example, the distillation feed stream can be fed to the distillation column between the rectifying section and the stripping section.

The distillation column can comprise a condenser, reflux drum, reboiler, gas distributor, reflux liquid distributor, feed tray, vacuum jacket, inner thermocouples located along a height of the distillation column, spiral-prismatic packing, automated flowrate control, automated temperature control, automated pressure control, automated level control, automated composition control, or a combination comprising at least one of the foregoing. The height of the packing layers can be adjusted to regulate the number of trays and their positions in the distillation column. The distillation column can comprise computer-controlled pumps. These pumps can control the distillation column parameters, for example, flowrates of streams entering and exiting the distillation column. The distillation column and related streams can be heated for example, using a Proportional-Integral-Derivative (PID) controlled electronic heater, and/or reboiler(s) (e.g., steam, oil, and/or electric).

The distillation column includes the designed amount of mass transfer devices (e.g., packing and/or trays) for separating the distillation feed stream. The distillation column can comprise a number of mass-transfer devices. For example, the distillation column can comprise 40 to 55 total equilibrium stages or theoretical trays equivalent to 54 to 75 total actual mass-transfer devices (e.g., trays). It is noted that 5 to 18 of the total equilibrium stages or theoretical trays (10 to 25 actual trays) can be located above the distillation feed stream, in the rectifying section of the distillation column.

A reflux ratio of the distillation column can be less than or equal to 1, for example, less than or equal to 0.8, for example, less than or equal to 0.5. Due to the present process, a reflux ratio of less than or equal to 0.40, for example, less than or equal to 0.2, or less than or equal to 0.1, such as 0 to 0.1, is possible. A reflux temperature for the distillation column can be 45 °C to 75 °C, for example, 49 °C to 50 °C. The distillation column can be operated at atmospheric pressure. The distillation column can also be operated under reduced pressure. Optionally, the distillation column can be operated at elevated pressure.

The distillation column (e.g., packed bed distillation column) can comprise a height "H" of total equilibrium stages or theoretical trays measured from a top portion of an uppermost equilibrium stage or theoretical tray (or other mass-transfer device) of the distillation column to a bottom portion of a bottommost equilibrium stage or theoretical tray (or other mass-transfer device) of the distillation column. The distillation column can further comprise temperature control points located on the distillation column at a percentage of H. For example, the location of the temperature control point can be a percentage of H represented by "% $H_i$" and determined by Equation (1):

$$\% \, H_i = (TT)_i \, / \sum TT \, (1),$$

wherein "$(TT)_i$" is an equilibrium stage or theoretical tray (or other mass transfer device equivalent to it) in the distillation column and "$\sum TT$" is a total number of equilibrium stages or theoretical trays in the distillation column +1.

A temperature profile for any temperature control point (any equilibrium stage or theoretical or real tray or other mass transfer device equivalent to it) can be calculated using Equation (2):

$$T(H) = C_1 H^3 + C_2 H^2 + C_3 H + C_4 \, (2),$$

wherein the equation is a piecewise polynomial function on the interval [0; 100] and "C" is a Cubic Hermite interpolant (for each sub-interval of T(H), the Cubic Hermite interpolant is provided in Table 5). The temperature at the temperature control point can be controlled by adjusting a temperature of the distillation feed stream, a reflux temperature of the distillation column, a reflux ratio of the distillation column, heat applied to the bottom portion of the distillation column, or a combination comprising at least one of the foregoing.

The distillation column can comprise one or more control points. For example, in accordance with Equation (1) and Equation (2), a first temperature control point can be located on the distillation column above the distillation feed stream inlet, in the rectifying section of the distillation column, at a height of 15% to 25% of H, wherein a temperature at the first temperature control point is 80 °C to 125 °C. A second temperature control point can be located on the distillation column below the distillation feed stream inlet, in the stripping section of the distillation column, at a height of 25% to 35% of H, wherein a temperature at the second temperature control point is 150 °C to 165 °C. A third temperature control point can be located on the distillation column below the distillation feed stream inlet, in the stripping section of the distillation column, at a height of 40% to 60% of H, wherein a temperature at the third temperature control point is 150 °C to 175 °C, for example, 165 °C to 170 °C. A fourth temperature control point can be located on the distillation column below the distillation feed stream inlet, in the stripping section of the distillation column, at a height of 65% to 90% of H, wherein a temperature at the fourth temperature control point is 160 °C to 180 °C, for example, 165 °C to 175 °C. When operating the distillation column under non-atmospheric pressure, the temperatures can be adjusted.

The distillation column can separate the distillation feed stream into products of phenol and acetone, by-products, and unreacted cumene for recycle. For example, the present method can include distributing a crude acetone fraction to a top portion of the distillation column and distributing a crude phenol fraction to a bottom portion of the distillation column. The crude acetone fraction can be withdrawn from the top portion of the distillation column. Optionally, at least a portion of the crude acetone fraction can be combined with the bypass acetone from the evaporator unit to form a combined crude acetone fraction. The crude acetone fraction from the distillation column can comprise less than or equal to 0.20 wt% of phenol, for example, less than or equal to 0.10 wt%, for example, less than or equal to 0.05 wt%, for example, less than or equal to 0.01 wt%.

A crude phenol fraction can be withdrawn from the bottom portion of the distillation column. The crude phenol fraction can comprise less than or equal to 25 ppm hydroxyacetone, for example, less than or equal to 10 ppm, for example, less than or equal to 5 ppm. The crude phenol fraction (e.g., due to the almost complete absence of hydroxyacetone) can comprise less than or equal to 10 ppm 2-methylbenofuran, for example, less than or equal to 5 ppm 2-methylbenofuran, for example, 0 ppm 2-methylbenofuran.

A temperature of the crude phenol fraction can be 181 °C to 190 °C, for example, 182 °C to 187 °C or 183 °C to 185 °C. The crude phenol fraction can be subjected to further downstream processing, for example, the production of high-quality phenol and then polycarbonates.

A more complete understanding of the components, processes, and apparatuses disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof and/or to define or limit the scope of the exemplary embodiments. Although specific terms are used in the following description for the sake of clarity, these terms are intended to refer only to the particular structure of the embodiments selected for illustration in the drawings and are not intended to define or limit the scope of the disclosure. In the drawings and the following description below, it is to be understood that like numeric designations refer to components of like function.

Referring now to FIG. 1, this schematic diagram represents an exemplary system configuration 10 used in a method for producing phenol and/or for separating phenol from a product stream. The system configuration 10 can include passing an evaporator unit feed stream 12 (e.g., phenol product stream 12) comprising phenol and acetone through an evaporator unit 14.

A bypass stream or bypass acetone 16 (e.g., comprising a part of a total light acetone fraction) can be withdrawn from a top portion 18 of the evaporator unit 14. A residual part of acetone fraction 42 (e.g., light acetone fraction 42, comprising a part of a total light acetone fraction) can be recycled back to a cleavage stage.

The system configuration 10 can include withdrawing a bottom fraction 22 from the evaporation unit 14, neutralizing the bottom fraction in neutralization unit 38 to form the distillation feed stream 20 comprising acetone and phenol, and passing the distillation feed stream 20 through a distillation column 24. The system configuration 10 can include distributing a crude acetone fraction to a top portion 28 of the column 24 and distributing a crude phenol fraction to a bottom portion 32 of the distillation column 24.

The distillation column 24 can comprise a height "H" of mass-transfer devices (e.g., equilibrium stages or theoretical trays or trays or structured packing) measured from a top portion of an uppermost mass transfer device of the distillation column 24 to a bottom portion of a bottommost mass transfer device of the distillation column 24. The distillation column 24 can further comprise a temperature control point 36 located on the distillation column 24 at a percentage of H.

An overhead stream emerging from the top 28 of the distillation column 24, after condensation in a condenser 40, can be divided into a distillate 26 which is obtained as the overhead product (e.g., the crude acetone fraction 26) and into a reflux (not shown) which can be returned back into the distillation column 24 and which has the same general composition as the distillate 26.

The distillate/crude acetone fraction 26 can be withdrawn from the condenser 40 and a phenol product stream 30 (e.g., crude phenol fraction 30) can be withdrawn from the bottom portion 32 of the distillation column 24. The crude acetone fraction 26, 34 is directed to acetone purification train/unit 44 and crude phenol fraction 30 is introduced to phenol purification train/unit 46. The system configuration 10 can include combining the bypass stream 16 and the crude acetone fraction 26 from the distillation column 24 forming a combined acetone stream 34, which can be directly directed to acetone purification train 44. It is noted that all or a portion of bypass stream 16 independent of its composition can be directed separately from crude acetone fraction 26 into a desired point of the acetone train 44.

The following examples are merely illustrative of the method of producing phenol disclosed herein and are not intended to limit the scope hereof.

EXAMPLES

Experimental trials were conducted using a continuous mode, laboratory-scale packed bed distillation column in accordance with the general system configuration 10 as seen in FIG. 1 for producing phenol and/or for separating phenol from a product stream. The distillation column was equipped with a vacuum jacket, five inner thermocouples located along the height of the distillation column, and spiral-prismatic packing. The height of the packing layers were adjusted to regulate the number of trays and their positions in the distillation column. The distillation column was also equipped with five automatic, computer-controlled pumps. These pumps were used to control the distillation column parameters, for example, flowrates of streams entering and exiting the distillation column. The distillation column and related streams were heated using a sand bath and a PID controlled electronic heater. After achievement of steady state (constant stream flowrates, stable temperature profile), parameters were varied, and multiple samples were taken at pre-determined time intervals. The samples were analyzed using HP-5890 Series II chromatographs.

Table 1 provides the main operating parameters for the distillation column and stream compositions for the feed stream (Feed), crude acetone stream, and crude phenol fraction (bottom fraction (BTM)). In Table 1, 80 (#49), 51 (#31), 31 (#18) and 17 (#9) are locations of temperature control points on the distillation column (percentage of height H and corresponding tray number in parenthesis). Corresponding temperatures are then listed below. The temperature of the crude phenol fraction was controlled by an external temperature controller. Table 2 provides feed compositions. In Table 1 and Table 2, Examples 1-6 and Comparative Examples 1-5 utilized raw feed materials taken from a full-scale phenol production plant. Examples 7-26 and Comparative Examples 6 and 7 utilized an artificial mixture wherein the cumene to water weight ratio was varied from 0.8 to 1.2 (Examples 7 to 17, 25, 26, Comparative Examples 6 and 7), from 1.20-1.45 (Examples 18 to 21) and from 1.45-1.70 (Examples 22 to 24). Artificial mixtures in Examples 18 to 21 and Examples 22 to 24 were prepared considering the preliminary selection of greater or equal to 25% and greater or equal to 50% of acetone bypass from the total recycle acetone stream from the evaporator flash unit, respectively.

The examples that utilized raw feed materials, and the examples for which the cumene to water weight ratio was varied from 0.8 to 1.2, were relatively unstable in the laboratory setting. For example, in Examples 1 and 2, with a feed rate equal to about 400 milliliters per hour (ml/hr), a desirable so called "determined" temperature profile (e.g., that provided improved results) was maintained and the hydroxyacetone content in the crude phenol fraction was 5.3 to 13.5 parts per million (ppm). In Comparative Examples 1 and 2, the determined temperature profiles were not maintained, and in Comparative Example 1, the distillation column flooded. In Examples 3 to 6 (the feed rate was increased to 466 to 484 milliliters per hour, a reflux ratio of 0 to 0.1 was maintained), a determined temperature profile for hydroxyacetone removal was maintained and the hydroxyacetone content in the crude phenol fraction was reduced to 2 to 18 ppm. In Comparative Examples 3 to 5, with a reflux ratio of 0.55 and 0 (e.g., without external reflux), the determined temperature profile was not maintained and was lower than desired, and the hydroxyacetone content in the crude phenol fraction increased to 65 to 614 ppm.

Examples 1 to 26 show that where a determined temperature profile was maintained, hydroxyacetone content in the crude phenol fraction generally does not exceed 20 ppm, such as less than 10 ppm, more particularly less than 5 ppm. If the crude phenol fraction (bottom stream) temperature is increased to 183.7 °C or higher and by slightly increasing temperature on Tray # 49 which is in the low part of the stripping section, it is possible to reduce the alpha-methylstyrene content in the crude phenol fraction to less than 0.20 weight percent (Examples 16 and 19), such as less than 0.10 weight percent (Examples 7, 9, 11 and 17). Simultaneously, the phenol content in the distillate does not exceed 0.10 weight percent (Examples 3, 7, and 8), such as less than 0.05 weight percent (Examples 1, 2, 4 to 6, and 9 to 17), when the cumene to water weight ratio is varied from 0.80 to 1.20.

Further, reducing the crude phenol fraction temperature lower than a temperature to increase alpha-methylstyrene removal does not affect the efficiency of hydroxyacetone removal, if the temperature in the remaining control points are matched to the determined temperature profile (Examples 1 to 6, 8, 10, 12 to 15, 18, and 20 to 26), but decreased the efficiency of alpha-methylstyrene removal. Reducing the temperature profile in the other control points different from the crude phenol fraction (Comparative Examples 1 to 6), as well as increasing the temperature (Comparative Example 7), leads to a decrease in efficiency of hydroxyacetone removal.

The data of Table 1 also shows that in Examples 7, 9, 11, 16, 17 and 19, in which a determined temperature profile for simultaneous HA and AMS removal was maintained, the HA content in the crude phenol stream did not exceed 5.5 ppm and AMS content was less than 0.2 wt%, such as less than or equal to 0.1 wt%. The main difference between the determined temperature profile for HA removal and the determined temperature profile for the simultaneous HA and AMS removal is that for the simultaneous removal of HA and AMS it can be appropriate to raise the temperature in the bottom stream and in the low part of the stripping section (e.g., on Tray # 49). However, a significant increase in temperature on Tray # 49 and Tray # 31, despite the almost complete removal of AMS, leads to the drop-down of HA (447 ppm of HA) into the bottom stream (Comparative Example 7).

| Table 1: Column operating parameters | | | | | | | | | |
|---|---|---|---|---|---|---|---|---|---|
| Example # (including comparative examples) | Feed rate (ml/hr) | Reflux ratio | Temperature (°C) | | | | | | |
| | | | Feed | Reflux | Crude phenol fraction (BTM) | Stripping (below feed) | | | Refining (above feed) |
| | | | | | | Height % (#Tray) | | | |
| | | | | | | 80(#49) | 51(#30) | 31(#18) | 17(#9) |
| 1 | 397.5 | 0.83 | 100.3 | 50.5 | 182.5 | 165.1 | 160.9 | 157.0 | 86.8 |
| Comp. 1 | 402.5 | 0.45 | 99.6 | 50.2 | 181.6 | 156.1 | 97.8 | 90.9 | 86.9 |
| Comp. 2 | 403.5 | 0.52 | 100.3 | 50.8 | 180.2 | 157.1 | 148.8 | 98.4 | 86.3 |
| 2 | 389.0 | 0 | 100.5 | - | 182.0 | 164.2 | 163.1 | 155.4 | 84.9 |
| Comp. 3 | 471.0 | 0 | 100.1 | - | 181.9 | 160.9 | 156.5 | 155.7 | 100.1 |
| Comp. 4 | 469.2 | 0.55 | 100.1 | 75.1 | 181.0 | 157.3 | 155.2 | 152.7 | 109.7 |
| Comp. 5 | 466.3 | 0.54 | 100.1 | 50.5 | 182.4 | 155.7 | 154.6 | 149.8 | 89.4 |
| 3 | 466.7 | 0 | 99.9 | - | 181.9 | 164.5 | 163.4 | 159.0 | 117.5 |
| 4 | 470.7 | 0 | 100.0 | - | 182.6 | 166.0 | 164.8 | 155.8 | 111.0 |
| 5 | 483.2 | 0 | 100.0 | - | 182.8 | 165.2 | 164.1 | 161.7 | 96.1 |
| 6 | 472.4 | 0 | 100.1 | 49.9 | 182.2 | 164.9 | 163.2 | 156.9 | 115.8 |
| Comp. 6 | 402.2 | 0.30 | 100.6 | 47.1 | 181.4 | 161.4 | 156.9 | 146.8 | 96.8 |
| 7 | 405.9 | 0 | 100.8 | - | 183.9 | 171.5 | 165.0 | 157.3 | 87.4 |
| 8 | 397.8 | 0.09 | 100.7 | 50.1 | 182.5 | 164.9 | 164.7 | 161.6 | 110.9 |
| 9 | 481.6 | 0 | 100.3 | - | 184.0 | 167.1 | 165.6 | 157.3 | 84.9 |
| Comp. 7 | 478.3 | 0.10 | 100.4 | 49.2 | 184.0 | 181.2 | 175.9 | 165.1 | 116.8 |
| 10 | 482.0 | 0.10 | 101.1 | 49.2 | 183.5 | 166.9 | 165.5 | 159.4 | 102.8 |

| Table 1: Column operating parameters | | | | | | | | | |
|---|---|---|---|---|---|---|---|---|---|
| Example # (including comparative examples) | Feed rate (ml/hr) | Reflux ratio | Temperature (°C) | | | | | | |
| | | | Feed | Reflux | Crude phenol fraction (BTM) | Stripping (below feed) | | | Refining (above feed) |
| | | | | | | Height % (#Tray) | | | |
| | | | | | | 80(#49) | 51(#30) | 31(#18) | 17(#9) |
| 11 | 529.0 | 0 | 100.3 | - | 183.9 | 166.9 | 165.6 | 159.1 | 112.6 |
| 12 | 534.2 | 0.10 | 100.5 | 49.1 | 183.2 | 167.0 | 166.6 | 160.9 | 113.6 |
| 13 | 484.3 | 0.10 | 109.7 | 49.2 | 182.7 | 166.2 | 165.7 | 162.0 | 116.8 |
| 14 | 530.0 | 0.10 | 110.8 | 49.3 | 181.9 | 166.4 | 165.9 | 161.7 | 110.8 |
| 15 | 483.9 | 0 | 90.2 | - | 182.9 | 166.2 | 164.9 | 155.8 | 90.2 |
| 16 | 482.5 | 0 | 90.3 | - | 183.8 | 166.1 | 165.2 | 160.3 | 108.2 |
| 17 | 491.0 | 0.10 | 85.5 | 49.6 | 183.8 | 171.3 | 164.3 | 159.9 | 106.1 |
| 18 | 481.6 | 0 | 100.6 | - | 182.1 | 166.7 | 165.7 | 157.2 | 112.0 |
| 19 | 480.6 | 0.10 | 100.5 | 48.9 | 184.0 | 166.0 | 165.8 | 160.4 | 120.8 |
| 20 | 481.3 | 0.11 | 100.6 | 49.3 | 183.2 | 166.0 | 165.7 | 160.7 | 120.3 |
| 21 | 531.1 | 0.10 | 100.1 | 50.1 | 183.5 | 167.9 | 165.1 | 161.8 | 121.0 |
| 22 | 481.4 | 0 | 100.4 | - | 183.3 | 165.9 | 165.5 | 162.2 | 126.8 |
| 23 | 483.7 | 0 | 100.0 | - | 182.7 | 166.8 | 166.4 | 161.3 | 126.0 |
| 24 | 481.1 | 0.10 | 100.2 | 48.9 | 182.8 | 166.5 | 166.0 | 162.8 | 131.0 |
| 25 | 401.3 | 0.24 | 100.8 | 50.4 | 182.2 | 164.9 | 164.7 | 161.7 | 117.4 |
| 26 | 395.1 | 0.51 | 100.2 | 49.7 | 182.1 | 165.3 | 164.3 | 157.7 | 114.3 |

| Table 1 (continued): Column operating parameters | | | | | | | | |
|---|---|---|---|---|---|---|---|---|
| Example # (including comparative examples) | Cumene to water mass ratio (feed stream) | Compositions | | | | | | |
| | | HA (ppm) | | | AMS (wt%) | | | Phenol in overhead (distillate) (wt%) |
| | | In Feed stream | In Crude phenol fraction | % Removed | In Feed stream | In Crude phenol fraction | % Removed | |
| 1 | 1.02 | 1197 | 5.3 | 99.6 | 4.17 | 1.69 | 59.4 | 0.04 |
| Comp. 1 | 1.11 | 1180 | 58.0 | 95.1 | 3.83 | 2.85 | 25.6 | 5.00 |
| Comp. 2 | 1.18 | 1380 | 75.5 | 94.5 | 4.28 | 5.81 | -35.7 | 0.61 |
| 2 | 0.88 | 1335 | 13.5 | 99.0 | 3.52 | 2.28 | 35.2 | 0 |
| Comp. 3 | 1.03 | 1359 | 613.5 | 54.9 | 4.38 | 2.93 | 33.1 | 0 |
| Comp. 4 | 1.03 | 1359 | 150.2 | 88.9 | 4.38 | 4.24 | 3.2 | 0 |
| Comp. 5 | 1.03 | 1359 | 65.1 | 95.2 | 4.38 | 1.99 | 54.6 | 0 |
| 3 | 1.19 | 1242 | 2.4 | 99.8 | 4.32 | 2.79 | 35.4 | 0.06 |
| 4 | 1.02 | 1197 | 17.7 | 98.5 | 4.17 | 1.56 | 62.6 | 0 |
| 5 | 1.16 | 1313 | 2.7 | 99.7 | 4.02 | 1.14 | 71.6 | 0.03 |
| 6 | 1.02 | 1197 | 3.6 | 99.7 | 4.17 | 2.32 | 44.4 | 0 |
| Comp. 6 | 1.02 | 1185 | 646 | 45.5 | 3.59 | 2.83 | 21.2 | 0.02 |
| 7 | 1.08 | 1349 | 5.5 | 99.6 | 2.02 | 0.01 | 99.5 | 0.07 |

(continued)

| Table 1 (continued): Column operating parameters | | | | | | | | |
|---|---|---|---|---|---|---|---|---|
| Example # (including comparative examples) | Cumene to water mass ratio (feed stream) | Compositions | | | | | | Phenol in overhead (distillate) (wt%) |
| | | HA (ppm) | | | AMS (wt%) | | | |
| | | In Feed stream | In Crude phenol fraction | % Removed | In Feed stream | In Crude phenol fraction | % Removed | |
| 8 | 1.10 | 1152 | 0 | 100.0 | 1.92 | 0.62 | 67.7 | 0.06 |
| 9 | 1.08 | 1349 | 0 | 100.0 | 2.02 | 0.10 | 95.0 | 0 |
| Comp. 7 | 1.08 | 1236 | 447.0 | 63.8 | 1.48 | 0.01 | 99.5 | 0.04 |
| 10 | 1.08 | 1349 | 0 | 100.0 | 2.02 | 0.58 | 71.3 | 0 |
| 11 | 1.08 | 1349 | 0 | 100.0 | 2.02 | 0.08 | 96.0 | 0 |
| 12 | 1.08 | 1236 | 0 | 100.0 | 1.98 | 1.42 | 28.3 | 0.05 |
| 13 | 0.97 | 1269 | 0 | 100.0 | 1.93 | 0.68 | 64.8 | 0 |
| 14 | 0.97 | 1269 | 1.4 | 99.9 | 1.93 | 1.18 | 38.9 | 0 |
| 15 | 1.00 | 1218 | 8.6 | 99.3 | 2.02 | 0.94 | 53.5 | 0 |
| 16 | 1.00 | 1218 | 0 | 100.0 | 2.02 | 0.19 | 90.6 | 0 |
| 17 | 1.09 | 1210 | 0 | 100.0 | 1.97 | 0.01 | 99.5 | 0.01 |
| 18 | 1.36 | 1342 | 1.7 | 99.9 | 2.19 | 2.50 | -14.2 | 0 |
| 19 | 1.31 | 1194 | 3.9 | 99.7 | 2.16 | 0.20 | 90.7 | 0.06 |
| 20 | 1.31 | 1194 | 5.1 | 99.6 | 2.16 | 1.30 | 39.8 | 0.06 |
| 21 | 1.31 | 1194 | 6.8 | 99.4 | 2.16 | 0.59 | 72.7 | 0.05 |
| 22 | 1.60 | 1276 | 4.3 | 99.7 | 2.38 | 1.42 | 40.3 | 0.19 |
| 23 | 1.63 | 1269 | 16.2 | 98.7 | 2.42 | 2.86 | -18.2 | 0.14 |
| 24 | 1.58 | 1339 | 1.7 | 99.9 | 2.37 | 2.61 | -10.1 | 0.14 |
| 25 | 1.10 | 1152 | 0 | 100.0 | 1.92 | 1.25 | 34.9 | 0.03 |
| 26 | 1.02 | 1962 | 4.5 | 99.8 | 3.70 | 3.62 | 2.2 | 0.06 |

| Table 2: Feed compositions | | | | | | | |
|---|---|---|---|---|---|---|---|
| Example # (including comparative examples) | Composition (wt%) | | | | | | Cumene to water mass ratio |
| | HA (ppm) | Cumene | AMS | Water | Acetone | Phenol | |
| Comp. 1 | 1180 | 10.46 | 3.83 | 9.42 | 27.98 | 44.98 | 1.11 |
| Comp. 2 | 1380 | 11.02 | 4.28 | 9.33 | 27.91 | 45.14 | 1.18 |
| 2 | 1335 | 9.81 | 3.52 | 11.08 | 28.19 | 45.11 | 0.88 |
| Comp. 3 - 5 | 1359 | 10.90 | 4.38 | 10.54 | 27.17 | 44.57 | 1.03 |
| 3 | 1242 | 11.22 | 4.32 | 9.44 | 27.30 | 45.08 | 1.19 |
| 1, 4, 6 | 1197 | 11.32 | 3.17 | 11.11 | 28.13 | 44.09 | 1.02 |
| 5 | 1313 | 10.85 | 4.02 | 9.39 | 27.90 | 45.17 | 1.16 |
| Comp. 6 | 1185 | 10.68 | 3.59 | 10.50 | 29.57 | 45.54 | 1.02 |
| 7,9-11 | 1349 | 11.62 | 2.02 | 10.73 | 29.49 | 46.02 | 1.08 |
| 8, 25 | 1152 | 11.51 | 1.92 | 10.46 | 29.88 | 46.11 | 1.10 |

(continued)

| Example # (including comparative examples) | Composition (wt%) | | | | | | Cumene to water mass ratio |
|---|---|---|---|---|---|---|---|
| | HA (ppm) | Cumene | AMS | Water | Acetone | Phenol | |
| Comp. 7 | 1236 | 11.52 | 1.98 | 10.53 | 29.56 | 46.02 | 1.08 |
| 12 | 1236 | 11.52 | 1.98 | 10.53 | 29.56 | 46.02 | 1.08 |
| 13, 14 | 1269 | 11.35 | 1.93 | 11.69 | 29.96 | 44.64 | 0.97 |
| 15, 16 | 1218 | 11.27 | 2.02 | 11.22 | 29.52 | 45.85 | 1.00 |
| 17 | 1210 | 11.44 | 1.97 | 10.49 | 29.28 | 46.70 | 1.09 |
| 18 | 1342 | 12.73 | 2.19 | 9.35 | 24.90 | 50.40 | 1.36 |
| 19-21 | 1194 | 12.46 | 2.16 | 9.54 | 25.19 | 50.53 | 1.31 |
| 22 | 1276 | 13.48 | 2.38 | 8.44 | 19.56 | 56.01 | 1.60 |
| 23 | 1269 | 13.70 | 2.42 | 8.40 | 19.35 | 56.00 | 1.63 |
| 24 | 1339 | 13.40 | 2.37 | 8.50 | 19.58 | 56.02 | 1.58 |
| 26 | 1962 | 10.75 | 3.70 | 10.48 | 30.08 | 44.79 | 1.02 |

Table 2: Feed compositions

The use of the acetone bypass stream or bypass acetone (16; FIG. 1) not only reduced the volumetric flow rate of the overhead stream of the distillation column (24; FIG. 1), but also allowed for an increase of the cumene to water weight ratio, a reduction of the acetone and water content of the feed stream, and an increase in the efficiency of the hydroxyacetone removal from the crude phenol fraction. At some phenol plants, at the decomposition stage of the CHP, to remove the heat of reaction, a recycle of acetone is used, during the evaporation of which the necessary removal of heat is provided. This recycle is formed in the evaporation unit and is a condensed vapor fraction containing mainly acetone. The bottom product from an evaporation unit is sent to the neutralization stage and then used as a feed to the distillation column to separate it into fractions. As a result, a quasi-stationary composition of the column feed is established in the system. When only a part of the recycle is taken (from the total recycle to the decomposition stage) and directed by a bypass around the distillation column directly to the acetone purification train in the feed of this column, first of all, the content of acetone and water decreases, the content of phenol and cumene increases, the remaining components change insignificantly (see Table 2 below). That is, using a recycle (bypass) leads to an increase in the weight ratio of cumene to water, an important parameter of feed affecting the efficiency removal of hydroxyacetone. For example, if in the initial feed (without recycle-bypass) the cumene to water weight ratio on average is about 1.0, then with the use of 25% and 50% recycle (bypass) this ratio increases on average to about 1.3 and 1.6, respectively. The use of greater or equal to 25 vol% and greater or equal to 50 vol% acetone bypass stream from the total recycle/bypass acetone stream from the evaporator flash unit (14; FIG. 1) reduced the volumetric flow rate of the overhead stream of the column (24; FIG. 1) by 8% and 17%, or reduced the volumetric flow rate of the distillation feed stream at least 6% and at least 13% respectively, due to decreasing mainly acetone content in the raw feed (20; FIG. 1) of the distillation column (24; FIG. 1). Simulation mass balances of the distillation column for separating the neutralized cleavage mixture in a conventional phenol plant can allow one to calculate changes in the main streams, for example, such as overhead (OVHD) and the feed caused using recycle (bypass) acetone from the evaporator unit around the distillation column directly to the acetone purification train. Simulations were carried out taking into account the feed rate reduction caused by the use of the set point recycle and constancy of the reflux ratio both without and with recycle. According to simulation (the calculation results), the use of 25% acetone recycle (from the total acetone recycle from the evaporation unit) in the form of a bypass allows one to reduce the overhead stream by at least 8.5% and at least 17% when using 50% acetone recycle from the evaporator unit.

Also, the cumene to water weight ratio was increased on average from 1.05 to 1.3 and 1.6, respectively. The maintenance of the determined temperature profile also allowed the lower reflux ratio to be used over a wide range of feed temperatures (e.g., 80 to 110 °C) and compositions (e.g., cumene to water weight ratio of 0.8 to 1.7).

Tables 3 and 4 show the dependency of the overhead crude acetone fraction (OVHD) on the feed flowrate of the distillation column (in which the cumene to water weight ratio varied from 0.8 to 1.20) and the reflux ratio. The distillation feed flowrate of the distillation column and the composition of the distillation feed stream did not have a significant effect on the overhead fraction, while the reflux ratio did have a significant effect on the overhead fraction (OVHD). The present configuration allowed for a reduction in the volumetric flow rate of the overhead stream/fraction of greater than or equal to 44%. The data in Tables 3 and 4 also demonstrates that if the reflux ratio ("R") is reduced while the flowrate of the crude acetone stream (OVHD) and the determined temperature profile are maintained, then the volumetric feed flowrate (F) can be increased by greater than or equal to 30%, thus allowing a significant increase in the capacity of the distillation column. Averaged data is provided in Table 3.

| Table 3 - Depending of overhead on reflux ratio | |
|---|---|
| OVHD $_{Rx}$ / OVHD $_{Ry}$ (x < y) | OVHD reduction, % |
| OVHD $_{R=0}$ / OVHD $_{R=0.8}$ | 44 |
| OVHD $_{R=0}$ / OVHD $_{R=0.5}$ | 30 |
| OVHD $_{R=0.1}$ / OVHD $_{R=0.5}$ | 24 |
| OVHD $_{R=0}$ / OVHD $_{R=0.25}$ | 17 |
| OVHD $_{R=0.1}$ / OVHD $_{R=0.25}$ | 8 |
| $OVHD = Distillate + Reflux$ | |

Reducing the reflux ratio from 0.5 (e.g., as used in an exemplary full-scale phenol production plant) to 0.1 and 0 (e.g., without external reflux), the volumetric flow rate of the overhead stream (OVHD) was reduced on average by 24 and 30%, respectively. Reducing the reflux ratio from 0.8 (e.g., as used in an exemplary full-scale phenol production plant) to 0, the volumetric flow rate of the overhead stream (OVHD) was reduced even more; up to 44%. Also, by reducing the reflux ratio from 0.25 to 0.1 and 0, this thereby reduced the volumetric flow rate of the overhead stream (OVHD) on average by 8 and 17%, respectively.

The data of Table 4 confirms that the feed rate increasing from 400 ml/hr (R = 0.5) to 480 ml/hr and then to 530 ml/hr with the simultaneous reducing of R from 0.5 to 0.1 or to 0 allows the reduction of OVHD from 334.8 ml/hr (400 ml/hr, R = 0.5) to 286.4 ml/hr (480 ml/hr, R = 0) and to 308 ml/hr (480 ml/hr, R = 0.1) and also to 317.8 ml/hr (530 ml/hr, R = 0) and to 329.9 ml/hr (530 ml/hr, R = 0.1) (e.g., to reduce OVHD by 8 to 14.5% (480 ml/hr) and 1.5 to 5.1% (530 ml/hr), respectively). Thus, reducing the reflux ratio from 0.5 to 0.1 and 0 with a constant load (OVHD flow rate) will allow an increase in the feed rate from about 400 ml/hr to about 480 ml/hr (an increase of 20%) and even up to about 530 ml/hr (an increase of 30%), but this is in the context of maintaining a determined temperature profile in the distillation column for increased efficiency of hydroxyacetone removal.

*"+" sign means that OVHD is increased*

| Table 4: Dependency of overhead stream (OVHD) on feed flowrate (F) and reflux ratio (R). | | | | | | |
|---|---|---|---|---|---|---|
| Ex. # | F (ml/hr) | R | OVHD flowrate (ml/hr) | OVHD/F | OVHD comparative ratio<br>$F_1(R_1)$ / $F_2(R_2)$<br>$F_1 > F_2$; $R_1 < R_2$ | OVHD reduction. % |
| 25 | 401.3 | 0.24 | 286.0 | 0.713 | | |
| 26 | 395.1 | 0.51 | 334.8 | 0.847 | | |
| 5 | 483.2 | 0 | 286.4 | 0.593 | $480_{(R=0)}$ / $400_{(R=0.5)}$ = 0.8554 | 14.5 |
| | | | | | $480_{(R=0)}$ / $400_{(R=0.25)}$ = 1.0014 | + 0.1 |
| 13 | 484.4 | 0.10 | 308.0 | 0.636 | $480_{(R=0.1)}$ / $400_{(R=0.5)}$ = 0.9200 | 8.0 |
| | | | | | $480_{(R=0.1)}$ / $400_{(R=0.25)}$ = 1.0769 | + 7.7 |
| 11 | 529.0 | 0 | 317.8 | 0.600 | $530_{(R=0)}$ / $400_{(R=0.5)}$ = 0.9492 | 5.1 |
| | | | | | $530_{(R=0)}$ / $400_{(R=0.25)}$ = 1.1112 | + 11.1 |
| 14 | 530.0 | 0.10 | 329.9 | 0.622 | $530_{(R=0.1)}$ / $400_{(R=0.5)}$ = 0.9854 | 1.5 |
| | | | | | $530_{(R=0.1)}$ / $400_{(R=0.25)}$ = 1.1534 | + 15.3 |

Taking into account too, the pre-selection of the part of the bypass acetone (e.g., light acetone fraction 16) from the evaporator flash unit (14; FIG. 1), and reducing the reflux ratio from 0.8 to 0.5 down to 0.1 and even down to 0 (without external reflux), while maintaining the determined temperature profile in the distillation column, this thereby allowed an increase in capacity (feed flow rate) of the distillation column of greater than or equal to 30%, for example greater than or equal to 40%, which significantly increased the production of phenol and acetone products.

Referring back to the data obtained in Tables 1 to 4, a determined temperature profile can comprise: (i) a first temperature control point located on the distillation column above the distillation feed stream inlet, in the rectifying section of the distillation column, at a height in a range of 15% to 19% of H (a total height "H" measured from a top portion of the distillation column to a bottom portion of the distillation column), wherein a temperature at the first temperature control point is 84 °C to 118 °C; and (ii) a second temperature control point located on the distillation column below the distillation feed stream inlet, in the stripping section of the distillation column, at a height of 28% to 34% of H, wherein a temperature at the second temperature control point is 155 °C to 165 °C; and (iii) a third temperature control point located on the distillation column below the distillation feed stream inlet in the stripping section of the distillation column, at a height of 46% to 56% of H, wherein a temperature at the third temperature control point is 160 °C to 168 °C; and (iv) a fourth temperature control point located on the distillation column below the distillation feed stream inlet, in the stripping section of the distillation column, at a height of 75% to 85% of H, wherein a temperature at the fourth temperature control point is 165 °C to 173 °C. The distillation column can be operated under atmospheric pressure with a feed temperature of 100 °C.

It is noted that for the simultaneous removal of hydroxyacetone and alpha-methylstyrene, the determined temperature profile is similar with the exception that the bottom temperature was increased up to 183.7 to 185 °C, and the temperature at the fourth temperature control point was also increased.

It is also noted that with increasing the total amount of mass-transfer devices in the distillation column, the locations of the control points are moving downwards relative to the distillation column; and with decreasing the total amount of mass-transfer devices in the distillation column, the locations of the control points are moving upwards relative to the distillation column.

It is also noted that, when the feed stream temperature was decreased from 110 °C to 85 °C, and the cumene to water weight ratio was varied from 0.8 to 1.2, the determined temperature profile in the bottom stream and at all control points did not experience any significant changes and showed only a slight reduction. Phenol content in the overhead stream (distillate) did not exceed 0.10 wt%, such as less than 0.05 wt%, more particularly about 0 %. When the cumene to water weight ratio in the feed stream was varied from 1.2 to 1.45, the determined temperature profile in the bottom stream and at the control points in the stripping section was essentially unchanged, whereas in the refining section the control point temperature was slightly raised on average to 116 to 126 °C. The phenol content in the distillate did not exceed 0.10 wt%, such as less than or equal to 0.06 wt%. When the cumene to water weight ratio in the feed stream was varied from 1.45 to 1.70, the determined temperature profile changed only in the refining section of the distillation column (above the distillation feed stream inlet, in the rectifying section of the distillation column); the control point at 15% to 19% H increased to 121 °C to 139 °C. The phenol content in the overhead stream was not significantly increased and did not exceed 0.20 wt%, such as less than 0.15 wt%.

FIG. 2 is a plotted line graph representing determined temperature profile data from a column 24 (FIG. 1) for use in a method of producing phenol and/or for separating phenol from a product stream. Six data sets were examined at a feed temperature of 100 °C. Accordingly, empirical equations were obtained which allow for the calculation of a determined temperature profile at any temperature control point on the column. The data sets are provided in Table 5. The determined temperature profile for removal of hydroxyacetone is shown in Data Sets 1 to 3. The determined temperature profile for removal of both hydroxyacetone and alpha-methylstyrene is shown in Data Sets 4 to 6. The cumene to water weight ratio was varied from 0.80 to 1.20 (Data Sets 1, 4), from 1.20 to 1.45 (Data Sets 2, 5), and from 1.45 to 1.70 (Data Sets 3, 6). Accordingly, the determined temperature profile for any temperature control point (any equilibrium stage or theoretical or real tray) can be calculated using the equation $T(H) = C_1H^3 + C_2H^2 + C_3H + C_4$, wherein the equation is a piecewise polynomial function on the interval [0; 100] and "C" is a Cubic Hermite interpolant. "H" is a total height measured from a top portion of the distillation column to a bottom portion of the distillation column. On each subinterval of T(H), the Cubic Hermite interpolant is provided in Table 5.

| Table 5: Temperature profile data | | | | |
|---|---|---|---|---|
| Data Set 1 | | | | |
| Subinterval | $C_1$ | $C_2$ | $C_3$ | $C_4$ |
| $0 \leq x < 0.6$ | - 43.3677 | 25.0064 | 18.4419 | 80.6000 |
| $0.6 \leq x < 17$ | 0.0055 | - 0.1524 | 1.6124 | 91.3000 |
| $17 \leq x < 31.4$ | - 0.0309 | 0.6513 | 1.0483 | 101.0000 |
| $31.4 \leq x < 51.2$ | 0.0002 | - 0.0191 | 0.6085 | 159.0000 |
| $51.2 \leq x < 79.6$ | 0.0001 | - 0.0049 | 0.0996 | 165.2000 |

(continued)

| Table 5: Temperature profile data | | | | |
|---|---|---|---|---|
| Data Set 1 | | | | |
| Subinterval | $C_1$ | $C_2$ | $C_3$ | $C_4$ |
| $79.6 \leq x \leq 100$ | - 0.0008 | 0.0489 | 0.1100 | 166.8000 |
| Data Set 2 | | | | |
| Subinterval | $C_1$ | $C_2$ | $C_3$ | $C_4$ |
| $0 \leq x < 0.6$ | - 82.5867 | 47.6498 | 34.3080 | 87.9000 |
| $0.6 \leq x < 17$ | 0.0071 | - 0.2050 | 2.2941 | 107.8000 |
| $17 \leq x < 31.4$ | - 0.0153 | 0.3071 | 1.2609 | 121.4000 |
| $31.4 \leq x < 51.2$ | 0.0002 | - 0.0179 | 0.5971 | 157.6000 |
| $51.2 \leq x < 79.6$ | 0.00015 | - 0.0062 | 0.1297 | 164.0000 |
| $79.6 \leq x \leq 100$ | - 0.0008 | 0.0502 | 0.1482 | 166.2000 |
| Data Set 3 | | | | |
| Subinterval | $C_1$ | $C_2$ | $C_3$ | $C_4$ |
| $0 \leq x < 0.6$ | - 93.1464 | 53.7390 | 38.7893 | 90.0000 |
| $0.6 \leq x < 17$ | 0.0078 | - 0.2327 | 2.6780 | 112.5000 |
| $17 \leq x < 31.4$ | - 0.0132 | 0.2514 | 1.3682 | 128.4000 |
| $31.4 \leq x < 51.2$ | 0.0001 | - 0.0127 | 0.4142 | 160.9000 |
| $51.2 \leq x < 79.6$ | 0.0001 | - 0.0037 | 0.0737 | 165.2000 |
| $79.6 \leq x \leq 100$ | - 0.0010 | 0.0549 | 0.0844 | 166.4000 |
| Table 5: Temperature profile data (continued) | | | | |
| Data Set 4 | | | | |
| Subinterval | $C_1$ | $C_2$ | $C_3$ | $C_4$ |
| $0 \leq x < 0.6$ | - 17.4505 | 10.0389 | 8.0922 | 86.4000 |
| $0.6 \leq x < 17$ | 0.0044 | - 0.1212 | 1.2923 | 91.1000 |
| $17 \leq x < 31.4$ | - 0.0299 | 0.6441 | 0.9034 | 99.3000 |
| $31.4 \leq x < 51.2$ | 0.0004 | - 0.0279 | 0.8345 | 156.5000 |
| $51.2 \leq x < 79.6$ | 0.0002 | - 0.0092 | 0.2166 | 165.3000 |
| $79.6 \leq x \leq 100$ | - 0.0006 | 0.0356 | 0.2399 | 169.2000 |
| Data set 5 | | | | |
| Subinterval | $C_1$ | $C_2$ | $C_3$ | $C_4$ |
| $0 \leq x < 0.6$ | - 79.9581 | 46.1498 | 32.7617 | 91.4000 |
| $0.6 \leq x < 17$ | 0.0058 | - 0.1648 | 1.7867 | 110.4000 |
| $17 \leq x < 31.4$ | - 0.0186 | 0.3834 | 1.0508 | 120.9000 |
| $31.4 \leq x < 51.2$ | 0.0003 | - 0.0195 | 0.5438 | 160.1000 |
| $51.2 \leq x < 79.6$ | 0.0002 | - 0.0069 | 0.1626 | 165.8000 |
| $79.6 \leq x \leq 100$ | - 0.0006 | 0.0391 | 0.1977 | 168.9000 |
| Data Set 6 | | | | |
| Subinterval | $C_1$ | $C_2$ | $C_3$ | $C_4$ |
| $0 \leq x < 0.6$ | - 90.5774 | 52.2250 | 38.6062 | 89.8000 |

(continued)

| Data Set 6 | | | | |
|---|---|---|---|---|
| Subinterval | $C_1$ | $C_2$ | $C_3$ | $C_4$ |
| $0.6 \leq x < 17$ | 0.0091 | - 0.2825 | 3.4526 | 112.2000 |
| $17 \leq x < 31.4$ | - 0.0093 | 0.1598 | 1.5332 | 133.0000 |
| $31.4 \leq x < 51.2$ | 0.0005 | - 0.0199 | 0.3818 | 160.6000 |
| $51.2 \leq x < 79.6$ | 0.00005 | 0.0010 | 0.2374 | 164.6000 |
| $79.6 \leq x \leq 100$ | - 0.0001 | 0.0088 | 0.3827 | 173.0000 |

The methods and processes disclosed herein include at least the following aspects:

Aspect 1: A method of separating phenol comprising separating a first portion of acetone from a product stream in an evaporator unit, wherein the first portion of the acetone includes recycle acetone and bypass acetone; recycling the recycle acetone; withdrawing a bottom fraction from the evaporator unit; neutralizing the bottom fraction to form a distillation feed stream that is directed to a distillation column; separating the distillation feed stream into a bottom stream and an overhead stream, the bottom stream including a crude phenol fraction; passing the overhead stream through a condenser to produce a distillate as a crude acetone fraction, and wherein a reflux ratio of the distillation column is less than or equal to 0.4, with the reflux ratio a ratio of a weight of a reflux to distillate weight; and directing the bypass acetone around the distillation column, wherein the product stream is a cumene hydroperoxide cleavage product stream; and wherein the recycle acetone is recycled to a cumene hydroperoxide cleavage.

Aspect 2: The method of Aspect 1, further comprising combining the crude acetone fraction with the bypass acetone to form a combined stream; purifying the combined stream to produce an acetone product; and purifying the crude phenol fraction to produce phenol.

Aspect 3: The method of any of the preceding aspects, wherein the bypass acetone comprises greater than or equal to 25 vol%, such as greater than or equal to 35 vol%, more particularly greater than or equal to 50 vol%, of the first portion of the acetone separated from the product stream.

Aspect 4: The method of any of the preceding aspects, wherein the reflux ratio of the distillation column is less than or equal to 0.2, such as less than or equal to 0.1, more particularly 0 to 0.1.

Aspect 5: The method of any of the preceding aspects, wherein the distillation feed stream comprises 10 wt% to 20 wt% of cumene or alpha-methylstyrene or combination thereof, such as 11 wt% to 16 wt% of cumene or alpha-methylstyrene or combination thereof; and wherein the distillation feed stream comprises 500 to 2,000 ppm hydroxyacetone, such as 1,000 to 1,400 ppm of hydroxyacetone.

Aspect 6: The method of any of the preceding aspects, wherein a temperature of the distillation feed stream is 60 °C to 120 °C, such as 75 °C to 110 °C, more particularly 100 °C.

Aspect 7: The method of any of the preceding aspects, wherein the distillation feed stream comprises a cumene to water weight ratio of 0.5 to 2.0, such as 0.8 to 1.7, more particularly 1.3 to 1.6.

Aspect 8: The method of any of the preceding aspects, wherein the crude acetone fraction comprises less than or equal to 0.20 wt% of phenol, such as less than or equal to 0.10 wt% of phenol, more particularly less than or equal to 0.05 wt% of phenol, even more particularly less than or equal to 0.01 wt% of phenol.

Aspect 9: The method of any of the preceding aspects, wherein the reflux for the distillation column has a temperature of 45 °C to 75 °C, such as 49 °C to 50 °C.

Aspect 10: The method of any of the preceding aspects, wherein the crude phenol fraction comprises less than or equal to 25 ppm hydroxyacetone, such as less than or equal to 10 ppm hydroxyacetone, more particularly less than or equal to 5 ppm; and wherein the crude phenol fraction comprises less than or equal to 0.20 wt% of alpha-methylstyrene, such as less than or equal to 0.10 wt% of alpha-methylstyrene, more particularly less than or equal to 0.05 wt% of alpha-methylstyrene, even more particularly less than or equal to 0.01 wt% of alpha-methylstyrene.

Aspect 11: The method of any of the preceding aspects, wherein a temperature of the crude phenol fraction is 181 °C to 190 °C, such as 182 °C to 187 °C, more particularly 183 °C to 185 °C.

Aspect 12: The method of any of the preceding aspects, wherein the volumetric flow rate of the overhead stream of the distillation column is reduced greater than or equal to 25%, such as greater than or equal to 35%, as compared to a distillation column having a reflux ratio of greater than or equal to 0.50; and wherein the volumetric flow rate of the distillation feed stream of the distillation column is increased greater than or equal to 30%, such as greater than or equal to 40%, as compared to a distillation column having a reflux ratio of greater than or equal to 0.50.

Aspect 13: The method of Aspect 1, further comprising directing the bypass acetone directly to an acetone purification stage.

Aspect 14: The method of any of the preceding aspects, wherein the distillation column further comprises 40 to 55 total equilibrium stages or theoretical trays equivalent to 55 to 75 total actual trays and wherein 5 to 20 of the total equilibrium stages or theoretical trays equivalent to 10 to 25 actual trays are located above an inlet of the distillation feed stream in a rectifying section.

Aspect 15: The method of any of the preceding aspects, wherein the distillation column further comprises a total height "H" measured from a top portion of the distillation column to a bottom portion of the distillation column; a first temperature control point located above an inlet for the distillation feed stream at a height 15% to 25% of H, wherein a temperature at the first temperature control point is 80 °C to 125 °C; a second temperature control point located below the inlet at a height 25% to 35% of H, wherein a temperature at the second temperature control point is 150 °C to 165 °C; a third temperature control point located below the inlet at a height 40% to 60% of H, wherein a temperature at the third temperature control point is 150 °C to 175 °C, such as, 165 °C to 170 °C; and a fourth temperature control point located below the inlet at a height 65% to 90% of H, wherein a temperature at the fourth temperature control point is 160 °C to 180 °C, such as, 165 °C to 175 °C.

Aspect 16: The method of any of the preceding aspects, further comprising maintaining a determined temperature profile along a section of the distillation column.

Aspect 17: The method of Aspect 17, wherein the determined temperature profile is adjusted by at least one of: adjusting composition of the distillation feed stream, such as adjusting composition of the distillation feed stream to a cumene to water weight ratio of 1.1 to 1.6; adjusting temperature of the distillation feed stream, such as from 95 to 105 °C; adjusting temperature of the reflux, such as from 49 to 51 °C; adjusting reflux ratio, such as less or equal to 0.1; or adjusting heat applied to a bottom of the distillation column.

Aspect 18: The method of Aspect 1, wherein a temperature of the distillation feed stream is 90 to 110 °C; wherein the distillation feed stream comprises a cumene to water weight ratio of 0.8 to 1.7; and further comprising maintaining a determined temperature profile along a section of the distillation column, and the temperature of a temperature control point for the distillation column is represented by the piecewise polynomial function T(H) on the interval [0; 100] and on each subinterval "T(H)" is a Cubic Hermite interpolant: $T(H) = C_1 H^3 + C_2 H^2 + C_3 H + C_4$, wherein "$C_1$" and "$C_2$" and "$C_3$" and "$C_4$" are coefficients for each subinterval.

Aspect 19: The method of Aspect 1, wherein the distillation column comprises a temperature control point and a height "H" of total equilibrium stages measured from a top portion of an uppermost equilibrium stage of the distillation column to a bottom portion of a bottommost equilibrium stage of the distillation column, wherein a location of the temperature control point is a percentage of H represented by "% Hi" and determined by the equation: $\% H_i = (TT)_i / \Sigma TT$; wherein "$(TT)i$" is an equilibrium stage and "$\Sigma TT$" is a total number of equilibrium stages in the column +1.

Aspect 20: The method of any of the preceding aspects, wherein the distillation column further comprises 54 to 75 trays, wherein 10 to 25 trays are located: (i) above an inlet of the distillation feed stream, and (ii) in a rectifying section.

Aspect 21: The method of any of the preceding aspects, wherein the distillation column is operated at atmospheric pressure.

Aspect 22: A method of producing high quality phenol, comprising: separating a light acetone fraction as a top fraction from a flash evaporator unit at a cumene hydroperoxide cleavage stage; directing a first portion of the light acetone fraction as bypass acetone around the distillation column directly to an acetone purification train; returning a residue part of the light acetone fraction as a recycle acetone fraction to the cumene hydroperoxide cleavage stage; withdrawing a bottom fraction from the flash evaporator unit; neutralizing the bottom fraction; directing the neutralized bottom fraction to a distillation column as a distillation feed stream; separating in the distillation column the distillation feed stream into an overhead stream and a bottom stream, the overhead stream comprising a crude acetone fraction and the bottom stream comprising a crude phenol fraction; directing the crude acetone fraction to the acetone purification train for producing an acetone product; directing the crude phenol fraction to a phenol purification train for producing high quality phenol; operating the distillation column with a reflux ratio of less than or equal to 0.4, such as less than or equal to 0.1, with the reflux ratio a ratio of reflux weight to distillate weight; operating the distillation column with a determined temperature profile and wherein the distillation column comprises a temperature control point and a height "H" of total equilibrium stages measured from a top portion of an uppermost equilibrium stage of the distillation column to a bottom portion of a bottommost equilibrium stage of the distillation column, wherein a temperature of a temperature control point is represented by the piecewise polynomial function T(H) on the interval [0; 100] and on each subinterval "T(H)" is a Cubic Hermite interpolant:

$$T(H) = C_1 H^3 + C_2 H^2 + C_3 H + C_4,$$

wherein "C" are coefficients for each subinterval;
wherein the crude phenol fraction comprises less than or equal to 25 ppm hydroxyacetone, such as less than or equal to 10 ppm hydroxyacetone, more particularly less than or equal to 5 ppm; and wherein the crude phenol fraction comprises less than or equal to 0.20 wt% of alpha-methylstyrene, such as less than or equal to 0.10 wt% of alpha-methylstyrene, more particularly less than or equal to 0.05 wt% of alpha-methylstyrene, even more particularly less than or equal to 0.01 wt% of alpha-methylstyrene.

Aspect 23: The method of Aspect 22, wherein the determined temperature profile allows for improved reduction of the reflux ratio and improved removal of hydroxyacetone or simultaneously hydroxyacetone and alpha-methylstyrene removal, and the removal is adjusted by the distillation feed stream and/or the reflux temperatures and/or by the reflux ratio and/or by heat applied to the bottom of the distillation column.

In general, the present disclosure may alternately comprise, consist of, or consist essentially of, any appropriate components herein disclosed. The present disclosure may additionally, or alternatively, be formulated so as to be devoid, or substantially free, of any components, materials, ingredients, adjuvants or species used in the prior art compositions or that are otherwise not necessary to the achievement of the function and/or objectives of the present disclosure. The endpoints of all ranges directed to the same component or property are inclusive and independently combinable (e.g., ranges of "less than or equal to 25 wt%, or 5 wt% to 20 wt%," is inclusive of the endpoints and all intermediate values of the ranges of "5 wt% to 25 wt%," etc.). Disclosure of a narrower range or more specific group in addition to a broader range is not a disclaimer of the broader range or larger group. "Combination" is inclusive of blends, mixtures, alloys, reaction products, and the like. Furthermore, the terms "first," "second," and the like, herein do not denote any order, quantity, or importance, but rather are used to denote one element from another. The terms "a" and "an" and "the" herein do not denote a limitation of quantity and are to be construed to cover both the singular and the plural, unless otherwise indicated herein or clearly contradicted by context. "Or" means "and/or." The suffix "(s)" as used herein is intended to include both the singular and the plural of the term that it modifies, thereby including one or more of that term (e.g., the film(s) includes one or more films). Reference throughout the specification to "one embodiment", "another embodiment", "an embodiment", and so forth, means that a particular element (e.g., feature, structure, and/or characteristic) described in connection with the embodiment is included in at least one embodiment described herein, and may or may not be present in other embodiments. In addition, it is to be understood that the described elements may be combined in any suitable manner in the various embodiments.

The modifier "about" used in connection with a quantity is inclusive of the stated value and has the meaning dictated by the context (e.g., includes the degree of error associated with measurement of the particular quantity). The notation "± 10%" means that the indicated measurement can be from an amount that is minus 10% to an amount that is plus 10% of the stated value. The terms "front", "back", "bottom", and/or "top" are used herein, unless otherwise noted, merely for convenience of description, and are not limited to any one position or spatial orientation. Unless defined otherwise, technical and scientific terms used herein have the same meaning as is commonly understood by one of skill in the art to which this present disclosure belongs. A "combination" is inclusive of blends, mixtures, alloys, reaction products, and the like.

**Claims**

1. A method of separating phenol comprising:

   separating a first portion of acetone from a product stream in an evaporator unit, wherein the first portion of the acetone includes recycle acetone and bypass acetone;
   recycling the recycle acetone;
   withdrawing a bottom fraction from the evaporator unit;
   neutralizing the bottom fraction to form a distillation feed stream that is directed to a distillation column;
   separating the distillation feed stream into a bottom stream and an overhead stream, the bottom stream including a crude phenol fraction;
   passing the overhead stream through a condenser to produce a distillate as a crude acetone fraction, and wherein a reflux ratio of the distillation column is less than or equal to 0.4, with the reflux ratio a ratio of a weight of a reflux to distillate weight; and
   directing the bypass acetone around the distillation column,
   wherein the product stream is a cumene hydroperoxide cleavage product stream; and
   wherein the recycle acetone is recycled to a cumene hydroperoxide cleavage stage.

2. The method of Claim 1, further comprising

combining the crude acetone fraction with the bypass acetone to form a combined stream;
purifying the combined stream to produce an acetone product; and
purifying the crude phenol fraction to produce phenol.

3. The method of any of the preceding claims, wherein the bypass acetone comprises greater than or equal to 25 vol%, such as greater than or equal to 35 vol%, more particularly greater than or equal to 50 vol%, of the first portion of the acetone separated from the product stream.

4. The method of any of the preceding claims, wherein the reflux ratio of the distillation column is less than or equal to 0.2, such as less than or equal to 0.1, more particularly 0 to 0.1.

5. The method of any of the preceding claims, wherein the distillation feed stream comprises 10 wt% to 20 wt% of cumene or alpha-methylstyrene or combination thereof, such as 11 wt% to 16 wt% of cumene or alpha-methylstyrene or combination thereof; and
wherein the distillation feed stream comprises 500 to 2,000 ppm hydroxyacetone, such as 1,000 to 1,400 ppm of hydroxyacetone.

6. The method of any of the preceding claims, wherein a temperature of the distillation feed stream is 60 °C to 120 °C, such as 75 °C to 110 °C, more particularly 100 °C.

7. The method of any of the preceding claims, wherein the distillation feed stream comprises a cumene to water weight ratio of 0.5 to 2.0, such as 0.8 to 1.7, more particularly 1.3 to 1.6.

8. The method of any of the preceding claims, wherein the crude acetone fraction comprises less than or equal to 0.20 wt% of phenol, such as less than or equal to 0.10 wt% of phenol, more particularly less than or equal to 0.05 wt% of phenol, even more particularly less than or equal to 0.01 wt% of phenol.

9. The method of any of the preceding claims, wherein the reflux for the distillation column has a temperature of 45 °C to 75 °C, such as 49 °C to 50 °C.

10. The method of any of the preceding claims, wherein the crude phenol fraction comprises less than or equal to 25 ppm hydroxyacetone, such as less than or equal to 10 ppm hydroxyacetone, more particularly less than or equal to 5 ppm; and
wherein the crude phenol fraction comprises less than or equal to 0.20 wt% of alpha-methylstyrene, such as less than or equal to 0.10 wt% of alpha-methylstyrene, more particularly less than or equal to 0.05 wt% of alpha-methylstyrene, even more particularly less than or equal to 0.01 wt% of alpha-methylstyrene.

11. The method of any of the preceding claims, wherein a temperature of the crude phenol fraction is 181 °C to 190 °C, such as 182 °C to 187 °C, more particularly 183 °C to 185°C.

12. The method of any of the preceding claims, wherein the volumetric flow rate of the overhead stream of the distillation column is reduced greater than or equal to 25%, such as greater than or equal to 35%, as compared to a distillation column having a reflux ratio of greater than or equal to 0.50; and
wherein the volumetric flow rate of the distillation feed stream of the distillation column is increased greater than or equal to 30%, such as greater than or equal to 40%, as compared to a distillation column having a reflux ratio of greater than or equal to 0.50.

13. The method of Claim 1, further comprising directing the bypass acetone directly to an acetone purification stage.

14. The method of any of the preceding claims, wherein the distillation column further comprises 40 to 55 total equilibrium stages or theoretical trays equivalent to 55 to 75 total actual trays and wherein 5 to 20 of the total equilibrium stages or theoretical trays equivalent to 10 to 25 actual trays are located above an inlet of the distillation feed stream in a rectifying section.

15. The method of any of the preceding claims, wherein the distillation column further comprises:

a total height "H" measured from a top portion of the distillation column to a bottom portion of the distillation column;

a first temperature control point located above an inlet for the distillation feed stream at a height 15% to 25% of H, wherein a temperature at the first temperature control point is 80 °C to 125 °C;

a second temperature control point located below the inlet at a height 25% to 35% of H, wherein a temperature at the second temperature control point is 150 °C to 165 °C;

a third temperature control point located below the inlet at a height 40% to 60% of H, wherein a temperature at the third temperature control point is 150 °C to 175 °C, such as, 165 °C to 170 °C; and

a fourth temperature control point located below the inlet at a height 65% to 90% of H, wherein a temperature at the fourth temperature control point is 160 °C to 180 °C, such as, 165 °C to 175 °C.

16. The method of any of the preceding claims, further comprising maintaining a determined temperature profile along a section of the distillation column.

17. The method of Claim 16, wherein the determined temperature profile is adjusted by at least one of:

adjusting composition of the distillation feed stream, such as adjusting composition of the distillation feed stream to a cumene to water weight ratio of 1.1 to 1.6;

adjusting temperature of the distillation feed stream, such as from 95 to 105 °C;

adjusting temperature of the reflux, such as from 49 to 51 °C;

adjusting reflux ratio, such as less or equal to 0.1; or

adjusting heat applied to a bottom of the distillation column.

18. The method of Claim 1, wherein a temperature of the distillation feed stream is 90 to 110 °C;

wherein the distillation feed stream comprises a cumene to water weight ratio of 0.8 to 1.7; and further comprising maintaining a determined temperature profile along a section of the distillation column, and the temperature of a temperature control point for the distillation column is represented by the piecewise polynomial function T(H) on the interval [0; 100] and on each subinterval "T(H)" is a Cubic Hermite interpolant:

$$T(H) = C_1 H^3 + C_2 H^2 + C_3 H + C_4,$$

wherein "$C_1$" and "$C_2$" and "$C_3$" and "$C_4$" are coefficients for each subinterval.

19. The method of Claim 1, wherein the distillation column comprises a temperature control point and a height "H" of total equilibrium stages measured from a top portion of an uppermost equilibrium stage of the distillation column to a bottom portion of a bottommost equilibrium stage of the distillation column, wherein a location of the temperature control point is a percentage of H represented by "% Hi" and determined by the equation:

$$\% \, H_i = (TT)_i \, / \sum TT,$$

wherein "$(TT)i$" is an equilibrium stage and "$\Sigma \, TT$" is a total number of equilibrium stages in the column +1.

**Patentansprüche**

1. Ein Verfahren zur Abtrennung von Phenol, das Folgendes umfasst:

das Abtrennen eines ersten Acetonanteils von einem Produktstrom in einer Verdampfereinheit, wobei der erste Acetonanteil Recycling-Aceton und Bypass-Aceton einschließt;

das Recyceln des Recycling-Acetons;

das Entnehmen einer Bodenfraktion aus der Verdampfereinheit;

das Neutralisieren der Bodenfraktion, um einen Destillations-Zustrom zu erzeugen, der zu einer Destillationskolonne geleitet wird;

das Auftrennen des Destillations-Zustroms in einen Bodenstrom und einen Kopfstrom, wobei der Bodenstrom eine Phenol-Rohfraktion einschließt;

das Durchleiten des Kopfstroms durch einen Kondensator, um ein Destillat als Aceton-Rohfraktion herzustellen, wobei ein Rücklaufverhältnis der Destillationskolonne weniger als oder gleich 0,4 ist, wobei das Rücklaufverhältnis ein Verhältnis eines Gewichts eines Rücklaufs zum Destillatgewicht ist; und
das Leiten des Bypass-Acetons um die Destillationskolonne herum,
wobei der Produktstrom ein Cumenhydroperoxid-Spaltungs-Produktstrom ist und
wobei das Recycling-Aceton zu einer Cumenhydroperoxid-Spaltungsstufe recycelt wird.

2. Das Verfahren gemäß Anspruch 1, das weiter Folgendes umfasst:

das Kombinieren der Aceton-Rohfraktion mit dem Bypass-Aceton, um einen kombinierten Strom zu erzeugen;
das Reinigen des kombinierten Stroms, um ein Acetonprodukt zu erzeugen; und
das Reinigen der Phenol-Rohfraktion, um Phenol zu erzeugen.

3. Das Verfahren gemäß einem beliebigen der obigen Ansprüche, worin das Bypass-Aceton mindestens 25 Volumenprozent, z. B. mindestens 35 Volumenprozent, genauer mindestens 50 Volumenprozent, des ersten Acetonanteils umfasst, der aus dem Produktstrom abgetrennt wird.

4. Das Verfahren gemäß einem beliebigen der obigen Ansprüche, worin das Rücklaufverhältnis der Destillationskolonne höchstens 0,2, z. B. höchstens 0,1, genauer 0 bis 0,1, beträgt.

5. Das Verfahren gemäß einem beliebigen der obigen Ansprüche, worin der Destillations-Zustrom 10 bis 20 Gewichtsprozent Cumen oder Alpha-Methylstyrol oder eine Kombination davon umfasst, z. B. 11 bis 16 Gewichtsprozent Cumen, Alpha-Methylstyrol oder eine Kombination davon; und
worin der Destillations-Zustrom 500 bis 2.000 ppm Hydroxyaceton, z. B. 1.000 bis 1.400 ppm Hydroxyaceton, umfasst.

6. Das Verfahren gemäß einem beliebigen der obigen Ansprüche, worin eine Temperatur des Destillations-Zustroms 60°C bis 120°C, z. B. 75°C bis 110°C, spezieller 100°C, beträgt.

7. Das Verfahren gemäß einem beliebigen der obigen Ansprüche, worin der Destillations-Zustrom ein Gewichtsverhältnis von Cumen zu Wasser von 0,5 bis 2,0, z. B. 0,8 bis 1,7, genauer 1,3 bis 1,6, umfasst.

8. Das Verfahren gemäß einem beliebigen der obigen Ansprüche, worin die Aceton-Rohfraktion höchstens 0,20 Gewichtsprozent Phenol, z. B. höchstens 0,10 Gewichtsprozent Phenol, genauer höchstens 0,05 Gewichtsprozent Phenol, noch genauer höchstens 0,01 Gewichtsprozent Phenol, umfasst.

9. Das Verfahren gemäß einem beliebigen der obigen Ansprüche, worin der Rückfluss für die Destillationskolonne eine Temperatur von 45°C bis 75°C, z. B. 49°C bis 50°C, hat.

10. Das Verfahren gemäß einem beliebigen der obigen Ansprüche, worin die Phenol-Rohfraktion höchstens 25 ppm Hydroxyaceton, z. B. höchstens 10 ppm Hydroxyaceton, genauer höchstens 5 ppm, umfasst, und
worin die Phenol-Rohfraktion höchstens 0,20 Gewichtsprozent Alpha-Methylstyrol, z. B. höchstens 0,10 Gewichtsprozent Alpha-Methylstyrol, genauer höchstens 0,05 Gewichtsprozent Alpha-Methylstyrol, noch genauer höchstens 0,01 Gewichtsprozent Alpha-Methylstyrol, umfasst.

11. Das Verfahren gemäß einem beliebigen der obigen Ansprüche, worin eine Temperatur der Phenol-Rohfraktion 181°C bis 190°C, z. B. 182°C bis 187°C, genauer 183°C bis 185°C, beträgt.

12. Das Verfahren gemäß einem beliebigen der obigen Ansprüche, worin der Volumenstrom des Kopfstroms der Destillationskolonne mehr als *(sic)* 25% reduziert wird, z.B. mehr als 35%, verglichen mit einer Destillationskolonne mit einem Rücklaufverhältnis mit mindestens 0,50; und
worin der Volumenstrom des Destillations-Zustroms der Destillationskolonne mehr als oder gleich *(sic)* 30%, z. B. mehr als oder gleich 40%, erhöht wird, verglichen mit einer Destillationskolonne, die ein Rücklaufverhältnis von mindestens 0,50 hat.

13. Das Verfahren gemäß Anspruch 1, das weiter das Leiten des Bypass-Acetons direkt in eine Aceton-Reinigungsstufe umfasst.

14. Das Verfahren gemäß einem beliebigen der obigen Ansprüche, worin die Destillationskolonne weiter 40 bis 55 Theoretische Trennstufen insgesamt oder theoretische Böden, äquivalent zu 55 bis 75 tatsächlichen Böden insgesamt, umfasst und worin 5 bis 20 der Theoretischen Trennstufen insgesamt oder der theoretischen Böden, äquivalent zu 10 bis 25 tatsächlichen Böden, sich oberhalb eines Einlasses des Destillations-Zustroms in einer Rektifikationszone befinden.

15. Das Verfahren gemäß einem beliebigen der obigen Ansprüche, worin die Destillationskolonne weiter Folgendes umfasst:

eine Gesamthöhe "H", gemessen von einem oberen Abschnitt der Destillationskolonne bis zu einem unteren Abschnitt der Destillationskolonne;
einen ersten Temperaturkontrollpunkt oberhalb eines Einlasses für den Destillations-Zustrom auf einer Höhe von 15% bis 25% von H, wobei eine Temperatur am ersten Temperaturkontrollpunkt 80°C bis 125°C beträgt;
einen zweiten Temperaturkontrollpunkt unterhalb des Einlasses auf einer Höhe von 25% bis 35% von H, wobei eine Temperatur am zweiten Temperaturkontrollpunkt 150°C bis 165°C beträgt;
einen dritten Temperaturkontrollpunkt unterhalb des Einlasses auf einer Höhe von 40% bis 60% von H, wobei eine Temperatur am dritten Temperaturkontrollpunkt 150°C bis 175°C, z. B. 165°C bis 170°C, beträgt; und
einen vierten Temperaturkontrollpunkt unterhalb des Einlasses auf einer Höhe von 65% bis 90% von H, wobei eine Temperatur am vierten Temperaturkontrollpunkt 160°C bis 180°C, z. B. 165°C bis 175°C, beträgt.

16. Das Verfahren gemäß einem beliebigen der obigen Ansprüche, das weiter das Aufrechterhalten eines festgelegten Temperaturprofils entlang einem Abschnitt der Destillationskolonne umfasst.

17. Das Verfahren gemäß Anspruch 16, worin das festgelegte Temperaturprofil durch mindestens eines von Folgendem angepasst wird:

Einstellen der Zusammensetzung des Destillations-Zustroms, z.B. Einstellen der Zusammensetzung des Destillations-Zustroms auf ein Gewichtsverhältnis von Cumen zu Wasser von 1,1 bis 1,6;
Einstellen der Temperatur des Destillations-Zustroms, z. B. von 95 bis 105°C;
Einstellen der Temperatur des Rücklaufs, z. B. von 49 bis 51°C;
Einstellen des Rücklaufverhältnisses, z. B. auf weniger als oder gleich 0,1; oder
Einstellen der Wärme, die auf einen Boden der Destillationskolonne einwirkt.

18. Das Verfahren gemäß Anspruch 1, worin eine Temperatur des Destillations-Zustroms 90 bis 110°C beträgt; worin der Destillations-Zustrom ein Gewichtsverhältnis von Cumen zu Wasser von 0,8 bis 1,7 umfasst; und weiter Folgendes umfassend:

das Aufrechterhalten eines festgelegten Temperaturprofils entlang einem Abschnitt der Destillationskolonne, und die Temperatur eines Temperaturkontrollpunkts für die Destillationskolonne durch die stückweise Polynomfunktion T(H) für das Intervall [0; 100] und für jedes Teilintervall "T(H)" dargestellt ist ein Cubic Hermite-Interpolant ist *(sic)* ;

$$T(H) = C_1H^3 + C_2H^2 + C_3H + C_4,$$

worin "$C_1$", "$C_2$", "$C_3$" und "$C_4$" Koeffizienten für jedes Teilintervall sind.

19. Das Verfahren gemäß Anspruch 1, worin die Destillationskolonne einen Temperaturkontrollpunkt und eine Höhe "H" von Theoretischen Trennstufen insgesamt umfasst, gemessen von einem oberen Abschnitt einer obersten Theoretischen Trennstufe der Destillationskolonne bis zu einem unteren Abschnitt einer untersten Theoretischen Trennstufe der Destillationskolonne, wobei eine Position des Temperaturkontrollpunkts ein Prozentsatz von H ist, dargestellt durch "% Hi" und festgelegt durch die Gleichung:

$$\% \ Hi = (TT)_i / \Sigma \ TT,$$

worin "$(TT)i$" eine Theoretische Trennstufe ist und "$\Sigma TT$" eine Gesamtzahl Theoretischer Trennstufen in der Kolonne +1 ist.

**Revendications**

1.  Procédé de séparation du phénol, comprenant :

    la séparation d'une première partie d'acétone d'un flux de produit dans une unité d'évaporation, où la première partie d'acétone comprenant de l'acétone de recyclage et de l'acétone de dérivation ;
    le recyclage de l'acétone de recyclage ;
    le prélèvement d'une fraction de fond de l'unité d'évaporation ;
    la neutralisation de la fraction de fond pour former un flux d'alimentation de distillation qui est dirigé vers une colonne de distillation ;
    séparer le flux d'alimentation de distillation en un flux de fond et un flux de tête, le flux de fond comprenant une fraction de phénol brut ;
    faire passer le flux de tête à travers un condenseur pour produire un distillat tel qu'une fraction d'acétone brute, et où le rapport de reflux de la colonne de distillation est inférieur ou égal à 0,4, le rapport de reflux étant le rapport entre le poids du reflux et le poids du distillat ; et
    diriger l'acétone de dérivation autour de la colonne de distillation,

    où le flux de produit est un flux de produit de clivage de l'hydroperoxyde de cumène ; et
    où l'acétone de recyclage est recyclée vers une étape de clivage de l'hydroperoxyde de cumène.

2.  Procédé selon la revendication 1, comprenant en outre combiner la fraction d'acétone brute avec l'acétone de dérivation pour former un flux combiné ;
    purifier le flux combiné pour produire un produit d'acétone ; et purifier la fraction de phénol brut pour produire du phénol.

3.  Procédé selon quelconque des revendications précédentes, où l'acétone de dérivation comprend plus de ou égal à 25 % en volume, tel que plus de ou égal à 35 % en volume, plus particulièrement plus de ou égal à 50 % en volume, de la première partie de l'acétone séparée du flux de produit.

4.  Procédé selon quelconque des revendications précédentes, où le rapport de reflux de la colonne de distillation est inférieur ou égal à 0,2, tel qu'inférieur ou égal à 0,1, plus particulièrement de 0 à 0,1.

5.  Procédé selon quelconque des revendications précédentes, où le flux d'alimentation de distillation comprend 10 % en poids à 20 % en poids de cumène ou d'alpha-méthylstyrène ou d'une combinaison de ceux-ci, tel que 11 % en poids à 16 % en poids de cumène ou d'alpha-méthylstyrène ou d'une combinaison de ceux-ci ; et
    où le flux d'alimentation de distillation comprend 500 à 2 000 ppm d'hydroxyacétone, tel que 1 000 à 1 400 ppm d'hydroxyacétone.

6.  Procédé selon quelconque des revendications précédentes, où la température du flux d'alimentation de distillation est 60 °C à 120 °C, tel que 75 °C à 110 °C, plus particulièrement 100 °C.

7.  Procédé selon quelconque des revendications précédentes, où le flux d'alimentation de distillation présente un rapport pondéral cumène/eau compris de 0,5 à 2,0, tel que 0,8 à 1,7, plus particulièrement 1,3 à 1,6.

8.  Procédé selon quelconque des revendications précédentes, où la fraction d'acétone brute comprend moins de ou égal à 0,20 % en poids de phénol, tel que moins de ou égal à 0,10 % en poids de phénol, plus particulièrement moins de ou égal à 0,05 % en poids de phénol, et plus particulièrement encore moins de ou égal à 0,01 % en poids de phénol.

9.  Procédé selon quelconque des revendications précédentes, où le reflux de la colonne de distillation a une température de 45 °C à 75 °C, tel que 49 °C à 50 °C.

10. Procédé selon quelconque des revendications précédentes, où la fraction de phénol brut comprend moins de ou égal à 25 ppm d'hydroxyacétone, tel que moins de ou égal à 10 ppm d'hydroxyacétone, plus particulièrement moins de ou égal à 5 ppm ; et
    où la fraction de phénol brut comprend moins de ou égal à 0,20 % en poids d'alpha-méthylstyrène, par exemple moins de ou égal à 0,10 % en poids d'alpha-méthylstyrène, plus particulièrement moins de ou égal à 0,05 % en poids d'alpha-méthylstyrène, et plus particulièrement encore moins de ou égal à 0,01 % en poids d'alpha-méthylstyrène.

**11.** Procédé selon quelconque des revendications précédentes, où la température de la fraction de phénol brut est 181 °C à 190 °C, tel que 182 °C à 187 °C, plus particulièrement 183 °C à 185 °C.

**12.** Procédé selon quelconque des revendications précédentes, où le débit volumétrique du flux de tête de la colonne de distillation est réduit plus de ou égal à 25 %, tel que plus de ou égal à 35 %, par rapport à une colonne de distillation ayant un rapport de reflux d'au moins 0,50 ; et
où le débit volumétrique du flux d'alimentation de la colonne de distillation est augmenté plus de ou égal à 30 %, tel que plus ou égal à 40 %, par rapport à une colonne de distillation ayant un rapport de reflux de plus de ou égal à 0,50.

**13.** Procédé selon la revendication 1, comprenant en outre l'acheminement de l'acétone de dérivation directement vers un étage de purification d'acétone.

**14.** Procédé selon quelconque des revendications précédentes, où la colonne de distillation comprend en outre 40 à 55 étages d'équilibre total ou plateaux théoriques équivalents à 55 à 75 plateaux réels au total, et où 5 à 20 des étages d'équilibre total ou plateaux théoriques équivalents à 10 à 25 plateaux réels sont situés au-dessus d'une entrée du flux d'alimentation de distillation dans une section de rectification.

**15.** Procédé selon quelconque des revendications précédentes, où la colonne de distillation comprend en outre :

une hauteur totale « H » mesurée depuis une partie supérieure de la colonne de distillation jusqu'à une partie inférieure de la colonne de distillation ;
un premier point de contrôle de température situé au-dessus d'une entrée pour le flux d'alimentation de la distillation à une hauteur de 15 % à 25 % de H, où la température au premier point de contrôle de température est 80 °C à 125 °C ;
un deuxième point de contrôle de température situé en dessous de l'entrée, à une hauteur de 25 % à 35 % de H, où la température au niveau du deuxième point de contrôle de température est de 150 °C à 165 °C ;
un troisième point de contrôle de température situé en dessous de l'entrée, à une hauteur comprise 40 % à 60 % de H, où la température au troisième point de contrôle de température est 150 °C à 175 °C, tel que 165 °C à 170 °C ; et
un quatrième point de contrôle de température situé en dessous de l'entrée, à une hauteur 65 % à 90 % de H, où la température au quatrième point de contrôle de température est 160 °C à 180 °C, tel que 165 °C à 175 °C.

**16.** Procédé selon quelconque des revendications précédentes, comprenant en outre le maintien d'un profil de température déterminé le long d'une section de la colonne de distillation.

**17.** Procédé selon la revendication 16, où le profil de température déterminé est ajusté par au moins l'une des:

ajuster une composition du flux d'alimentation de la distillation, tel qu'ajuster la composition du flux d'alimentation de distillation à un rapport pondéral cumène/eau de 1,1 àt 1,6 ;
ajuster la température du flux d'alimentation de distillation, tel que de 95 à 105 °C ;
ajuster la température du reflux, tel que de 49 à 51 °C ;
ajuster le rapport de reflux, tel qu'inférieure ou égale à 0,1 ; ou
ajuster une chaleur appliquée au fond de la colonne de distillation.

**18.** Procédé selon la revendication 1, où la température du flux d'alimentation de distillation est 90 à 110 °C ;

où le flux d'alimentation de distillation comprend un rapport pondéral cumène/eau de 0,8 et 1,7 ; et comprenant en outre
le maintien d'un profil de température déterminé le long d'une section de la colonne de distillation, et la température d'un point de contrôle de température pour la colonne de distillation est représentée par la fonction polynomiale par morceaux T(H) sur l'intervalle [0 ; 100] et où, sur chaque sous-intervalle, « T(H) » est une interpolation d'Hermite cubique :

$$T(H) = C_1 H^3 + C_2 H^2 + C_3 H + C_4,$$

où « $C_1$ », « $C_2$ », « $C_3$ » et « $C_4$ » sont des coefficients pour chaque sous-intervalle.

**19.** Procédé selon la revendication 1, où la colonne de distillation comprend un point de contrôle de température et une hauteur « H » des étages d'équilibre totaux mesurée depuis une partie supérieure d'un étage d'équilibre le plus haut de la colonne de distillation jusqu'à une partie inférieure d'un étage d'équilibre le plus bas de la colonne de distillation, où l'emplacement du point de contrôle de température est un pourcentage de H représenté par « % Hi » et déterminé par l'équation :

$$\% \ H_i \ = \ (TT)_i \ / \ \Sigma \ TT,$$

où « (TT)i » est un étage d'équilibre et « $\Sigma$ TT » est le nombre total d'étages d'équilibre dans la colonne +1.

FIG. 1

**EP 4 237 397 B1**

**Patent documents cited in the description**

- RU 2020135296
- US 3405038 A
- US 4251325 A
- RU 2323202